# EUROPEAN PATENT SPECIFICATION

(11) **EP 1 802 974 B1**
(45) Date of publication and mention of the grant of the patent: **07.01.2009**
(21) Application number: 05806447.8
(22) Date of filing: 30.09.2005
(51) Int. Cl.: G01N 33/558

(54) **ANALYTICAL DEVICES WITH PRIMARY AND SECONDARY FLOW PATHS**
ANALYSEVORRICHTUNGEN MIT PRIMÄREN UND SEKUNDÄREN STRÖMUNGSWEGEN
DISPOSITIFS D'ANALYSE DOTES DE CANAUX D'ECOULEMENT PRIMAIRE ET SECONDAIRE

(30) Priority: 30.09.2004 US 615116 P
(43) Date of publication of application: 04.07.2007
(73) Proprietor: QUIDEL CORPORATION, San Diego, California 92121 (US)
(72) Inventor: LY, Peter, San Jose, CA 95133 (US); MEHRA, Rajesh, Sunnyvale, CA 94085 (US); PAWLAK, Catherine, San Jose, CA 95135 (US); MERIS, Romy, Livermore, CA 94550 (US); PAWLAK, Jan W., San Jose, CA 95135 (US)
(74) Representative: Price, Nigel John King
(86) International application number: PCT/US2005/035275
(87) International publication number: WO 2006/039542

(56) References cited:
- WO-A-97/06437
- US-A- 5 275 785
- US-A1- 2004 152 207
- US-B1- 6 436 722
- US-B1- 6 689 317

## Description

### BACKGROUND OF THE INVENTION

Ligand-receptor interactions (including, e.g., antibody-antigen interactions, nucleic acid hybridization, enzyme/enzyme substrate, small molecule/large molecule interactions and binding protein-nucleic acid interactions) can be detected using steps that accomplish: (1) introducing a liquid sample into a device, (2) tagging at least a portion of an analyte present in the sample by reacting the sample with a binding reagent (referred to herein as a "tag") for the analyte; (3) contacting the sample containing any tagged analyte with an analyte capturing reagent, thereby removing at least a portion of the tagged analyte of interest from the bulk of un-reacted sample; (4) removing unreacted species (e.g., unreacted tag or analyte) from the region of the device containing the analyte capture reagent; and (5) detecting and/or analyzing the presence of the captured analyte in the region containing the analyte capture reagent.

One class of devices for conducting such receptor-ligand assays uses porous material throughout the assay. Typically in these devices, all reagents (e.g., tagging reagents and capture reagents) are located on or in one or more porous materials that are in fluid communication with one another. Liquid sample flows through the porous materials and interactions between the sample and reagents occur therein. Typically, each reagent within these devices is localized to a specific zone on or in the porous material. Typically, liquid sample is introduced to the porous material and thereafter moves through the zones for example, by capillary migration. An example of this type of assay device is a lateral flow or strip assay. Such lateral flow devices may be comprised of more than one piece of porous material in which case the strips are typically sequentially aligned (frequently in overlapping relationship to one another) to form a single, substantially straight and continuous flow path within the device.

Exemplary lateral flow devices are described for example in U.S. Patent Nos: 4,943,522, Eisenger, et al., 5,096,837, Fan et al., 5,223,220, Fan et al., 5,521,102, Boehringer, et al., 5,766,961, Pawlak, et al. and 5,770,460, Pawlak, et al. Additional lateral flow devices are described, for example, in U.S. Patent Nos: 6,485,982, Charlton, et al., 6,352,862, Davis, et al., 5,622,871, May, et al., 5,656,503, May et al. and 6,534,320, Ching et al.

Typically, such lateral flow devices employ a visually detectable label, such as a metal sol or colored latex bead, to visualize captured analyte. However such labels must be present in the capture zone in sufficient quantity to be seen which in turn requires a minimum quantity of analyte to be present in the sample and captured in the capture zone. In contrast, enzyme linked immunosorbent assays (ELISAs), which employ enzyme-based label systems and are generally conducted in a plate format, are typically more sensitive than such lateral flow devices, but require multiple steps to accomplish the assay, in particular requiring sequential reactions and one or more washes between such reactions.

Thus, while both lateral flow and ELISA type ligand/receptor assay systems are useful, each have certain disadvantages. There is a need in the art, therefore, for devices that can provide flexibility with respect to choice of label in a one-step assay format, for example, by permitting for sequential reactions and/or fluid flow with single step functionality. The present invention addresses this and other needs.

Citation of documents herein is not intended as an admission that any is pertinent prior art. All statements as to the date or representation as to the contents of documents is based on the information available to the applicant and does not constitute any admission as to the correctness of the dates or contents of the documents.

US 6 689 317 B1 discloses an immunoassay analytical test apparatus comprising a zone for receiving a sample containing an analyte, a zone for receiving a mobile phase, a detection means for permitting detection of the analyte by immunoreaction, a first flow path for flow of the analyte in the mobile phase from the sample receiving zone to the detection means, and a second flow path permitting flow of a mobile phase to the detection means.

US 2004/152207 A1 discloses a 1-step enzyme immunoassay in which enzyme/antibody conjugate or label and enzyme substrate are separated until separation of bound and free enzyme conjugate or label is complete. This separation is accomplished by using variable flow paths, immobilisation of substrate at the test line, placement of substrate in a sac or association with a particle layer, enzyme product chemical capture, delay zone dissolution and protected enzyme substrates.

US 6 436 722 B1 discloses a device for performing an assay comprising two separate flow paths established sequentially in the device with a single user activation step. A first flow path is configured to deliver a sample and conjugate soluble binding reagents to a solid phase. A second flow path is configured to allow a wash reagent to remove unbound conjugate and sample from the solid phase to an absorbent and optionally deliver detection reagents. Sample/conjugate mixture in the first flow path is prevented from entering the second flow path because capillary and surface energy of the second flow path prevent it from being wetted by this mixture.

### BRIEF SUMMARY OF THE INVENTION

The present invention provides analytical devices and methods for performing an assay to determine the presence or approximate quantity of at least one analyte in a liquid sample. The scope of the invention is defined in the appended claims. In some embodiments, described herein, the secondary flow path is comprised of non-porous material and the primary flow path is comprised of porous and non-porous materials. Some devices of the present invention further comprise a tagging zone located in the primary flow path, upstream of the capture zone and downstream of the junction of the secondary and primary flow paths.

In some embodiments of the present invention, the device further comprises an absorbent (absorptive material) downstream of and in fluid communication with the capture zone, wherein withdrawal of liquid from the secondary flow path into the primary flow path is facilitated by absorption of the liquid by the absorptive material.

In some embodiments of the devices described herein, one or more reagents are located in the secondary flow path. Exemplary reagents include without limitation, label reagents (such as, enzyme substrates), conditioning reagents, control reagents, end-of-assay reagents and/or reference reagents.

In some exemplary embodiments described herein, the tagging zone is associated with a non-porous material and the capture zone is associated with a porous material.

In some embodiments of the devices described herein, the tag is linked to a detectable label. In some embodiments of the devices described herein, the tag is linked to a visually-detectable label. In some embodiments of the devices described herein, the tag is linked to an enzyme, which when contacted with an enzyme substrate, converts the enzyme substrate into a detectable label. In some embodiments of the devices described herein, the secondary flow path comprises the enzyme substrate. Advantageously, such devices provide a true one-step enzyme-based immunoassay.

In some embodiments of the devices described herein, the tag comprises a first linking member. And a label, located in the secondary flow path, comprises a detectable moiety and a second linking member coupled thereto, wherein the first linking member and second linking member are capable of binding or otherwise associating with one another. In some embodiments, the detectable moiety is a colored moiety. In some embodiments, one of the first linking member and second linking member is biotin and the other is (strept)avidin.

In some embodiments of the devices described herein, the device comprises at least two secondary flow paths in fluid communication with the primary flow path, wherein each secondary flow path forms only one junction with the primary flow path and the tagging zone is located downstream from the junctions. In such embodiments each of the secondary flow paths may comprise the same or different reagents or no reagents at all.

In some embodiments of present invention, the devices further comprise a control zone located in the primary flow path. Particularly where the device is used to detect a single analyte in a liquid sample, the control zone in such device is preferably located downstream of the capture zone. As described elsewhere herein, the control zone preferably comprises an immobilizing control reagent capable of immobilizing the tag or a separate control reagent. Control reagents may be located in a primary or secondary flow path and may comprise a directly or indirectly detectable moiety. The control reagent is preferably substantially mobilizable when contacted with the liquid sample.

In some embodiments of the devices described herein, a control reagent comprising a detectable moiety, which reagent is substantially mobilizable when contacted with the liquid sample, is comprised within a secondary flow path. In such embodiments, the device further comprises a control zone located in the primary flow path downstream from the capture zone and comprises an immobilizing control reagent capable of binding the detectable control reagent such that a flow of the liquid sample from the primary flow path into the secondary flow path then back into the primary flow path through to the control zone can be ascertained.

In further embodiments of the present invention, the devices comprise an end-of-assay zone located in the primary flow path and downstream of the capture zone and, where present any control zones. The end-of-assay zone comprises a moiety capable of producing a detectable signal when contacted with the sample, thereby indicating the arrival of the sample to the end of assay zone and a suitable time to ascertain the test result.

In alternative embodiments of the devices described herein, the sample port is in fluid communication with a chamber at a level below the level of the tagging zone; and the chamber is in fluid communication with the flow path such that the sample enters the flow path from the bottom of the flow path between the secondary flow path and the tagging zone (i.e., the junction of the secondary flow path and primary flow path). In this embodiment, the liquid sample splits with a portion of the sample flowing downstream into contact with the tagging zone and a second portion flowing upstream into the secondary flow path (contacting any reagents located therein).

In still other embodiments of the present invention, the devices further comprise a housing for enclosing the flow path, the housing comprising any or all of:
i) a sample entry port for receiving an aqueous sample;
ii) one or more vents to facilitate desired sample flow; and
iii) reading access means for permitting the capture zone and/or control zone and/or end-of-assay zone to be read.

The present invention further provided methods for the detection of an analyte in a liquid sample employing the devices of the present invention. By way of example, in one embodiment, the method comprises applying a liquid sample to a device of the present invention comprising a capture zone and determining the presence or approximate quantity of an analyte in the capture zone.

In further embodiments of the present invention, provided are methods comprising applying a liquid sample to a device of the present invention such that a portion of the liquid sample flows through the primary flow path to a conditioning zone (if present) then to a tagging zone, wherein tag is mobilized and reacts with analyte present in the sample, then flows to the capture zone, wherein at least a portion of tagged analyte is captured, and then flows through any remaining zones to the end of the assay; and a second portion of the liquid sample flows into a secondary flow path of the device, wherein label is mobilized; and said second portion of liquid sample subsequently flows out of said secondary flow path and into said primary flow path to said capture zone, wherein said label reacts with captured tagged analyte to generate a visibly detectable signal indicative of the presence or approximate quantity of analyte present in the sample.

### BRIEF DESCRIPTION OF THE DRAWINGS

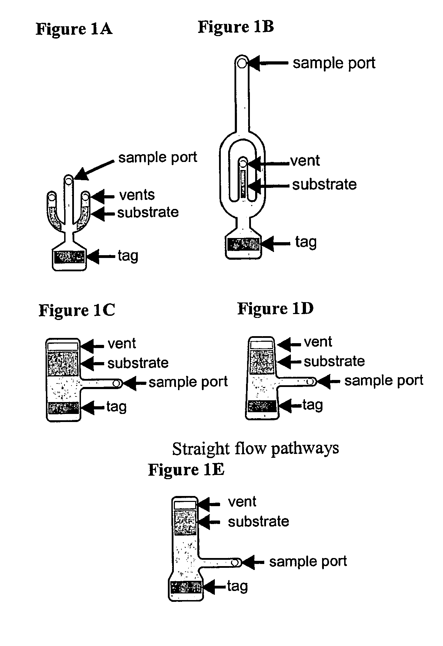

Figures 1A-E illustrate exemplary configurations of secondary and a portion of primary flow paths of analytical devices in accordance with the present invention. In particular are illustrated exemplary embodiments of a non-porous portion of devices in accordance herewith. In these figures, vents and a sample application site (sample entry port) are indicated as voids. Each of the pathways is intended to be in fluid communication with the capture zone (not shown) located downstream of the tag zone (at the bottom of each drawing). Figure 1A illustrates two secondary flow paths each branching from a different side of the primary flow path. Figure 1B illustrates a primary flow path splitting to pass along either side of the upstream end of a secondary flow path. In both Figures 1A and 1B sample initially flows directly downstream from the sample entry port with a portion of the sample thereafter flowing upstream out of the primary flow path and into the one or more secondary flow paths. Figures 1C, 1D and 1E illustrate embodiments in which the sample flows laterally from the sample entry port and then flows both upstream into the secondary flow path and downstream into the primary flow. As illustrated in the figures, these exemplary embodiments locate the tag and the substrate (an exemplary label component) in the non-porous structure. The capture zone, as well as the control and end-of-assay zones, if present, are located downstream of the region illustrated and, in some embodiments are located on or in a porous material.

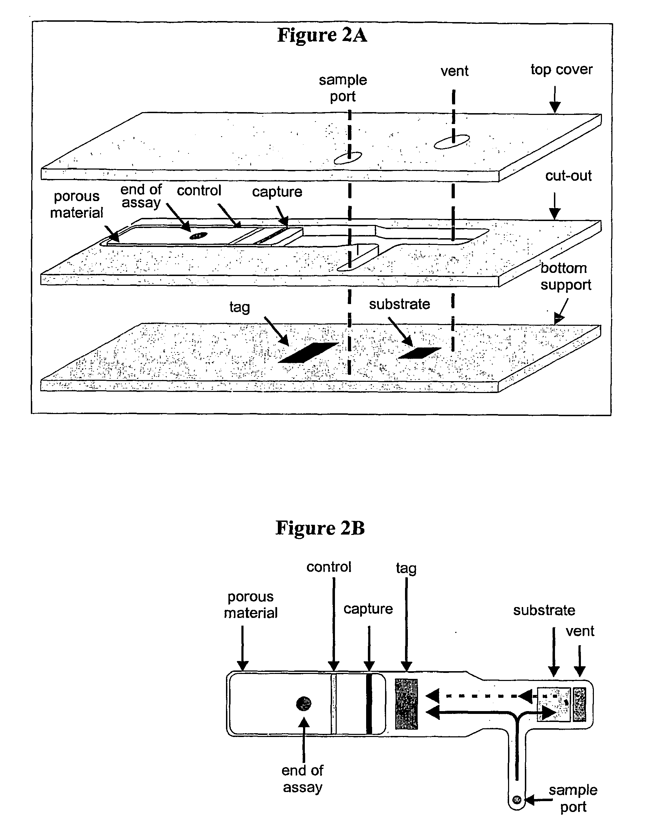

Figures 2-5 illustrate various flow path and reagent configurations for different embodiments of analytical devices in accordance with the present invention. Although no absorbent material, (typically positioned at the end of the assay and in fluid communication therewith), is shown in these figures, such is contemplated. By way of example, an absorbent material may be placed between the cut-out and top cover of Figure 2A, in fluid communication with the porous material but not overlapping any of the capture zone, control zone or end-of-assay zone.
Figure 2A illustrates an exemplary analytical device of the present invention comprising a non-porous portion of the flow path in fluid communication with a porous portion of the flow path, the non-porous portion having a configuration similar to the embodiments depicted in Figures 1C, D, and E. In this embodiment, the tagging zone is located in the non-porous portion of the device downstream of the junction of the primary and secondary flow paths. The top cover, cut-out and bottom support elements illustrated (as well as similar elements illustrated in other figures) are shaded for the purpose of providing contrast to the figures. It will be appreciated by those of skill in the art that the top cover in particular is preferably transparent and/or includes an additional cut-out above the capture, control and/or end-of-assay zones such that those zones may be observed by the user.
Figure 2B depicts a top view of the configuration of the porous/non-porous flow path portion of the embodiment depicted in Figure 2A, including positioning of reagents therein. This figure illustrates positioning of the secondary flow path upstream (to the right in the figure) of the sample entry port. In this embodiment, a portion of liquid sample flows from the sample entry port, first laterally then upstream, to contact the substrate. The remaining portion of the liquid sample flows from the sample entry port downstream into the primary flow path and towards the end of the assay. The solid arrow located within the non-porous portion of the device illustrates initial flow of the sample and the dashed line indicates delayed, subsequent flow from the secondary flow path into the primary flow path.

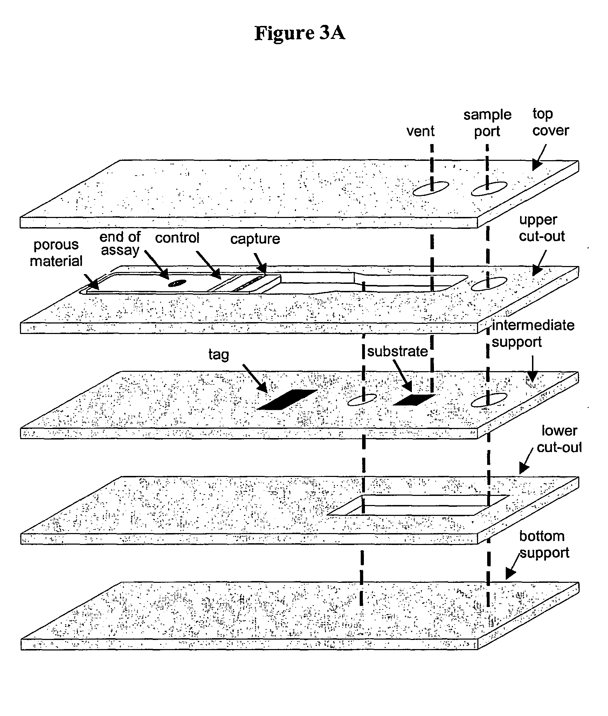

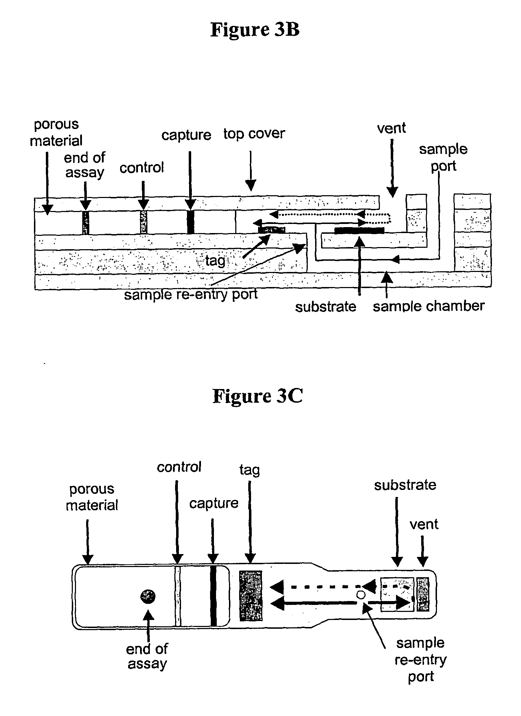

Figure 3A illustrates an exploded view of another embodiment of an analytical device in accordance herewith, the analytical portion of which is similar to that illustrated in Figure 2A. In this embodiment, however, two bottom layers, a bottom support and lower cut-out, are added to create a sample chamber below the secondary flow path. An intermediate support comprises cut-outs which provide for fluid communication between the sample chamber and the upper cut-out (analytical) portion of the device. Figure 3B provides a cross-section view of the assembled device illustrated in Figure 3A. In operation, liquid sample is applied to the sample port. The sample flows down through cut-outs in the upper cut-out and intermediate support and into the sample chamber. Once sample has filled the sample chamber, it flows through the sample re-entry port into the analytical portion of the device (the upper cut-out). Sample then flows both upstream into the secondary flow path and down stream into the primary flow path to provide sequential delivery of sample to the capture zone in accordance herewith. This may be accomplished in various ways, for example, downstream flow (i.e., flow from the sample port or channel towards the capture zone) can be driven by the capillary action of the porous component of the flow path, while upstream flow (i.e., flow from the sample port into the secondary flow path comprising, for example, enzyme substrate) can be driven by the capillary action of the non-porous hollow channel. Alternatively, sufficient liquid sample may be added to the sample port or channel to fill the non-porous hollow channel and contact the porous component of the flow path. In such an alternative, fluid flow out of the non-porous hollow channel occurs as the porous component absorbs the fluid. Thus, in either case, once there is no sample left at the entry port, then the porous component of the flow path draws the sample back from the secondary flow path comprising the enzyme substrate zone.
Figure 3C depicts a top view of the analytical portion (upper cut-out) of the device depicted in Figure 3A, with the top removed. As with Figure 2B, the solid-lined arrows within the flow paths indicate the initial flow of fluid and the dashed-lined arrows indicate the delayed, or secondary flow of fluid.

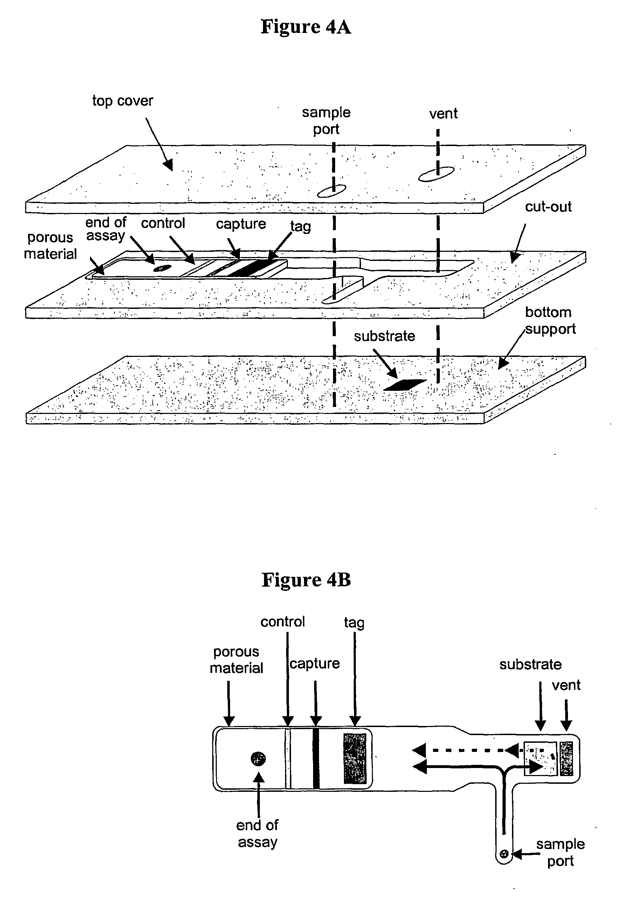

Figure 4A illustrates an exploded view of an alternative embodiment of a device in accordance with the present invention that is similar to the device illustrated in Figures 2A and 2B but wherein the tagging zone is located in or on a porous material rather than on a non-porous material. Figure 4B depicts a top view (looking through the top cover) of the analytical (or cut-out) portion of the embodiment depicted in Figure 4A. The solid arrows shown in the flow path illustrate initial flow of the sample both upstream into the secondary flow path and downstream into the primary flow path, and the dashed line arrows illustrate subsequent or delayed flow from the secondary flow path into the primary flow path.

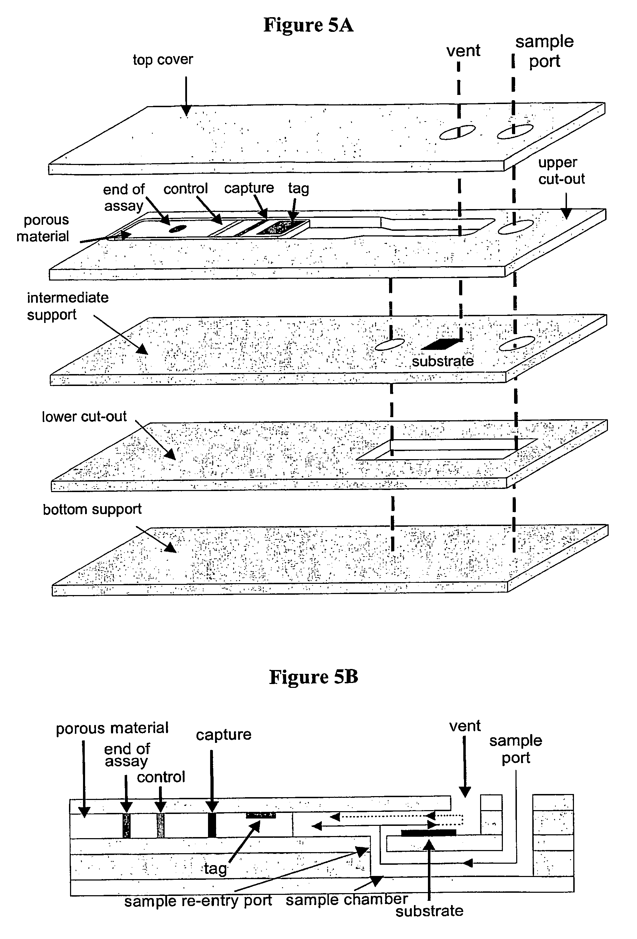

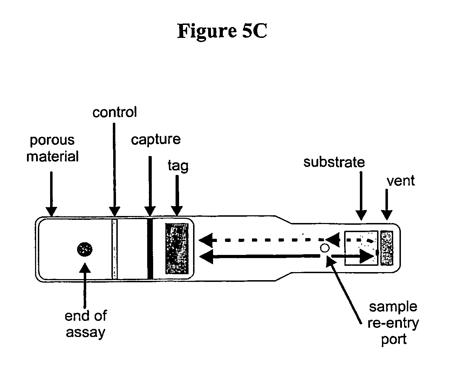

Figure 5A is an exploded view of a device similar to that illustrated in Figure 4A but wherein the tagging zone is located on or in a porous material rather than a non-porous material in the primary flow path. As with Figure 4A, two bottom layers are added to create a sample chamber located below the secondary flow path. Figure 5B provides a cross-section of the assembled device illustrated in Figure 5A. Similarly to Figure 4B, sample applied to the sample port flows down into the sample chamber entering the analytical portion (upper cut-out) of the device via the sample re-entry port. The sample then flows both upstream into the secondary flow path and downstream into the primary flow path. Subsequently, sample located in the secondary flow path flows into the primary flow path and through to the end of the assay. Figure 5C illustrates a top view, through the cover, of the analytical (top-cut-out) portion of the embodiment of Figure 5A. The solid arrows shown in the flow path illustrate initial flow of the sample both upstream into the secondary flow path and downstream into the primary flow path, and the dashed line arrows illustrate subsequent or delayed flow from the secondary flow path into the primary flow path.

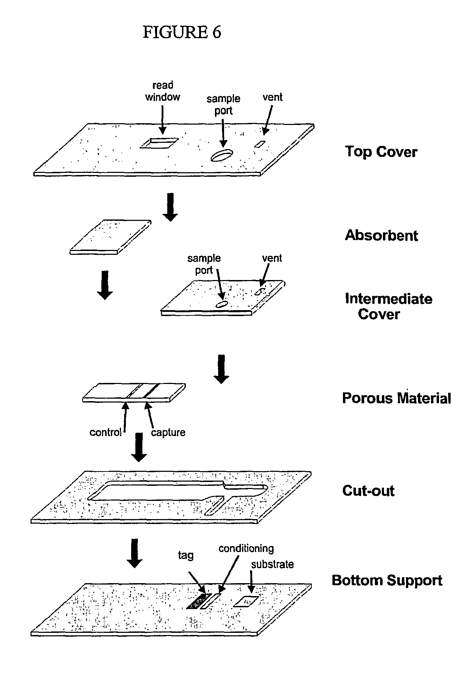

Figure 6 illustrates an exploded view of an analytical device in accordance with the present invention and exemplary assembly order thereof. In particular, illustrated is a non-porous bottom support having three reagents printed thereon; substrate (an exemplary label component), located in the secondary flow path, and conditioning reagent and tag, located in the primary flow path. To the top of this bottom, support, the cut-out is affixed. The porous material is placed within the cut-out where illustrated. An intermediate cover is located atop the cut-out with the vent located at the upstream end of the secondary flow path. An absorbent is placed atop of and in fluid communication with the porous material down stream of the control zone. Finally a top cover, preferably comprising cut-outs for sample port, vent and one or more read windows, is aligned atop and affixed to the intermediate cover and cut-out to form a complete device.

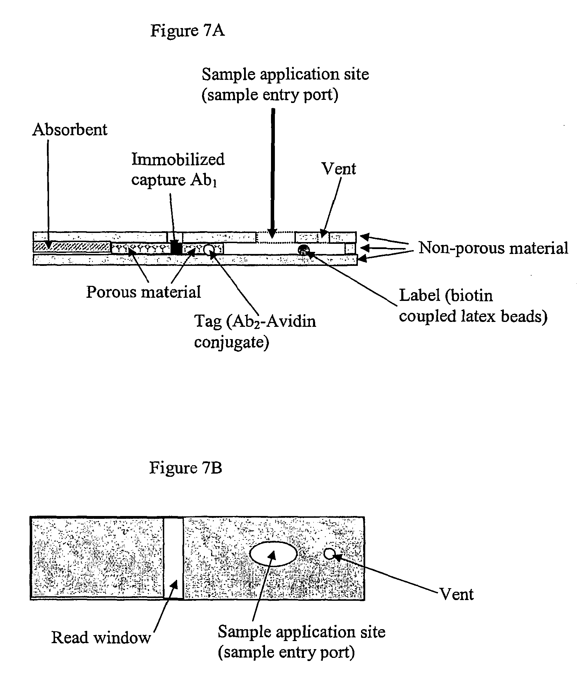

Figure 7A illustrates a cross-section of a further embodiment of an analytical device of the present invention employing a non-enzymatic label system and locating the tagging zone in or on a porous material. In this figure, a non-enzymatic label, located in the secondary flow path, comprises colored latex beads having biotin coupled thereto. The biotin-labeled beads are located on the non-porous material such that the label is mobilizable upon application of a fluid, such as the liquid sample. Downstream of the secondary flow path, in the primary flow path, a mobilizable tag is located on or in the porous material and comprises an antibody capable of binding the target analyte, which antibody is coupled with (strept)avidin. Downstream of and in fluid communication with the tagging zone, a second antibody, also capable of binding the target analyte, is immobilized on or in the porous material to form the capture zone. It will be appreciated by those of skill in the art that the tagging and capture zones may be located on the same or different porous materials, provided however if more than one piece of porous material is employed, the pieces will be located in fluid communication with one another.

During operation of the assay illustrated in Figure 7A, liquid sample is applied to the sample application site (or sample entry port) where a portion flows upstream into the secondary flow path, mobilizing the biotin coupled latex bead label, and the remaining portion flows downstream into the primary flow path. In the primary flow path, the sample enters the porous material, mobilizes the tag and, if present in the sample, analyte binds to the antibody (Ab₂)-avidin complex (tag). The analyte-Ab₂-avidin complex then flows with the sample to the capture zone where the capture antibody binds to, or captures, the analyte-antibody-avidin complex, forming a sandwich comprising capture antibody (Ab₁)-analyte-antibody tag (Ab₂-avidin). As liquid sample evacuates the primary flow path, that portion of the sample located in the secondary flow path enters the primary flow path bringing with it the mobilized label. Sample from the secondary flow path enters the porous material and contacts the control zone. The avidin portion of the captured tag then binds to or captures the biotin-latex bead label, the accumulation of which label in the capture zone results in a visually detectable signal indicative of the presence of analyte in the sample. Also shown in Figure 7A is an absorbent material located downstream of the capture zone which may serve both as a "sink" for the liquid sample and to facilitate flow through the analytical device.

Figure 7B illustrates a top-view of the exemplary device of Figure 7A, wherein the air vent for the secondary flow path is shown, along with the area or opening for sample application and a transparent viewing (or read) window, which is located above the capture zone, so one can see the test results.

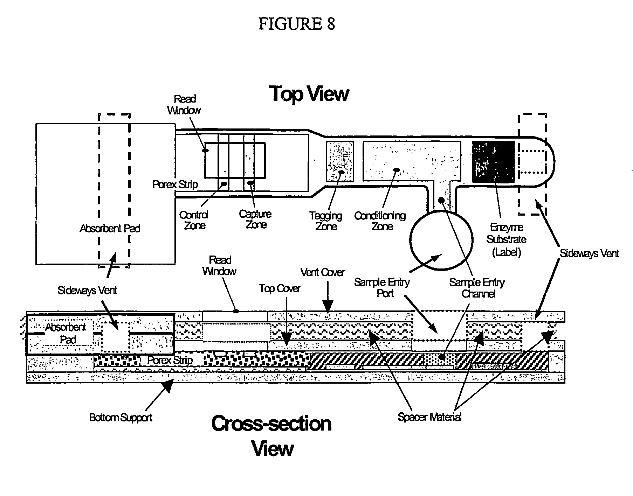

Figure 8 illustrates a top view and a cross-section view of an embodiment of an analytical device according to the present invention. A sample suspected of containing a target analyte is added to the sample entry port of the device and enters the sample entry channel. A portion of the liquid sample moves through the primary flow path to the conditioning zone, tagging zone, then to the capture and control zones. In this illustrated embodiment, the capture and control zones are located on or in a porous material, such as a Porex® strip, whereas the tagging and conditioning zones are located on a non-porous material.

As liquid sample flows through the primary flow path and contacts the tagging zone, the tag (in this illustration, comprising an enzyme) is mobilized and carried with the sample into the porous material and through the capture zone. If analyte is present in the sample, it will complex with the tag and be captured by the immobilized capture reagent located in the capture zone. Those of skill in the art will recognize that the tag and analyte may form a complex before or after being captured by the capture reagent. If no analyte is present in the sample, the tag will not be captured by the capture reagent but will continue to flow with the sample to the end of the assay.

As one portion of the sample flows through the primary flow path, the remaining sample flows into a non-porous secondary flow path. Located in the secondary flow path is the enzyme substrate which is mobilized by the sample. Advantageously, the sample within the secondary flow path re-enters the primary flow path subsequent to the initial sample flow into the primary flow path. Thus, the sample from the secondary flow path, comprising the enzyme substrate, flows through the porous material to the capture zone and contacts the immobilized tagged analyte, whereupon the tag (in this illustration, comprising an enzyme) reacts with the enzyme substrate to produce a detectable signal.

An absorbent pad at the end of the assay together with sideways air vents, as illustrated, facilitate flow of sample through the device. A sideways vent in the secondary flow path facilitates flow of sample into and out of the secondary flow path. In the particular embodiment illustrated, a read window is located above the control and capture zones in order to permit easy inspection of those zones by the user for determination of the results of the assay. An intermediary cover is located beneath the top cover and on top of the porous material and forming the top portion of the non-porous portion of the device. A bottom layer runs the length of the device.

It will be appreciated by those of skill in the art that the devices illustrated in these figures are intended to illustrate certain aspects and/or configurations of devices in accordance with the present invention. They are in no way intended to be limiting with respect to device configuration. By way of example, none of Figures 2 through 5 illustrate the optional absorbent material at the end of the assay that is illustrated in Figures 6 through 8. Such an absorbent may be included in the illustrated devices and such is likewise contemplated herein. Similarly, additional and/or different reagents and/or zones may be included in devices in accordance with the present invention than are necessarily illustrated in the figures.

### DETAILED DESCRIPTION OF THE INVENTION

### I. Definitions

"Analyte" refers to any substance that can be detected. For example, an analyte can be a component of a sample that is desirably retained and detected during the course of operating the analytical device described herein (e.g., a molecule, such as a protein, antigen or antibody, or a cell in a sample) or an analog or derivative of the component of a sample. Accordingly, the component of the sample may be detected directly or indirectly, such as by detecting a derivative, analog or other moiety that is predictive of the amount or presence of the component.

A "tag" refers to a detectable, mobilizable molecule that is capable of binding to analyte or derivative of thereof. Tags may be directly detectable, for example as in the case of a tag comprising a colored moiety, or they may be indirectly detectable, for example in the case of a tag comprising an enzyme.

"Label" refers to a detectable moiety, which results in a visual or otherwise detectable signal. Labels may be associated with an analyte or a derivative of an analyte either directly or indirectly through an analyte-specific tag or secondary complex of linking members. A tag may comprise a directly detectable label, for example, such as, an analyte-specific antibody coupled to a colored latex bead (the label) or other colored moiety, or a tag may comprise a label component, such as for example an enzyme which requires a substrate (second label component) to generate a detectable signal (such as, a visual signal). The term "label" as used herein includes single components of a label system, such as enzymes and substrates. Label can be colorless but capable of producing a detectable signal when reacted with another label component, such as for example, an enzyme acting on an enzyme substrate to generate a colored molecule. As used herein "colored moiety" includes, without limitation, colored particles, colored particulates and colored molecules. Exemplary colored moieties include, without limitation, metal sols, colored latex and/or chemical associations including colored molecules, such as enzyme/antibody/substrate/colored molecule associations.

A "linking member" refers to a molecule that interacts with, binds or otherwise associates with a second molecule in a mixture, other than the analyte. As used herein, a first and second linking member interact with each other. Examples of linking members include any two molecules with affinity for each other, e.g., biotin and streptavidin.

"Tagging zone" as used herein refers to a region in a device that comprises a tag. For example, the tagging zone can comprise an antibody capable of binding to the target analyte, wherein the antibody is associated with a label, such as for example a colored moiety, or with a label component, such as an enzyme.

"Capture zone" refers to a region in a device that comprises immobilized reagent ("capture reagent") capable of directly or indirectly binding an analyte or a derivative of an analyte to be detected. Typically, the capture reagent contacts, binds, or otherwise associates with an analyte, a derivative thereof, or a complex comprising an analyte or derivative thereof thereby retaining the analyte or complex in the capture zone. "Immobilized" in this context of immobilized capture reagent means that a sufficient amount of the capture reagent remains in the capture zone throughout the assay procedure to permit determination of the presence or approximate quantity of the analyte. Capture reagent does not have to be retained beyond the time needed to make such determination. Immobilization of the capture reagent in the capture zone may occur by, but is not limited to, covalent bonding or adsorption. Where the capture zone is located on and/or within a porous material, depending upon the nature of the material, derivatization to permit covalent bonding of the capture reagent in the capture zone, for example using glutaraldehyde or a carbodiimide, can be employed. It is not necessary that the capture reagent be bound directly, chemically, biologically or otherwise, to the porous material. The capture reagent may be attached to another material which other material is physically entrapped in the porous material to form the capture zone or is otherwise affixed in the capture zone by any physical, chemical or biochemical means. For example, capture reagents can be attached covalently or passively to beads or the like and the beads then affixed on or entrapped within the porous material or, if a non-porous material is employed affixed on the non-porous material. Those of skill in the art will readily understand and implement various methods or procedures for immobilizing capture reagent on or within the material comprising the capture zone.

A "derivative of an analyte" refers to a product resulting from a chemical or enzymatic modification of the analyte or any substance whose concentration in the sample is directly proportional to the analyte. For example, the derivative of an analyte may be a complex of the analyte with an additional component that, in turn, binds to the capture reagent, or with an additional component which serves merely to label the analyte, but does not interfere with the analyte's ability to bind to the capture reagent. In another illustration, the derivative of an analyte might be a reaction product formed in stoichiometric relationship to the analyte in a reaction, wherein the reaction product binds to the capture reagent.

"Downstream" refers to the direction, for example of fluid flow, away from the point of liquid application and toward the end of the assay..

"Upstream" refers to the direction, for example of fluid flow, away from rather than toward the end of the assay.

"Flow path" refers to a route taken by a fluid/liquid as it flows, whether in a housing, in or on a non-porous component or material and/or in or on a porous component or material. The flow path may be a single route or include several routes, where each route may support liquid flow simultaneously, sequentially or independently relative to other routes and where each route may or may not flow into one or more other routes. Flow paths include fluid passages, chambers, channels, conduits, matrices or other structures in which or through which fluids can travel. A "primary flow path" refers to a flow path that comprises fluid communication from the upstream end, where sample enters, downstream through the capture zone to the end of the assay. The primary flow path may further comprise one or more of a conditioning zone, tag zone, control zone and end-of-assay zone. As used herein, a "secondary flow path" refers to a flow path that forms a single junction with a primary flow path upstream of a capture zone, and wherein a portion of liquid applied to the device moves from the primary flow path into the secondary flow path and then returns to the primary flow path as the liquid in the primary flow path moves through the device. The secondary flow path may comprise porous or non-porous materials or both.

A "junction" refers to a location where at least two flow paths meet, thereby allowing fluid communication between the at least two flow paths. The junction can comprise a merger of two or more non-porous flow paths, two or more porous flow paths, a merger between one or more porous flow path and one or more non-porous flow path, or a bifurcation of one flow path into two or more flow paths.

A "porous" material or component refers to a water-insoluble material comprising pores through which liquid may flow. By way of example, a porous material may be comprised of a network of insoluble material that supports liquid flow by capillary force. Capillaries within typical porous materials are randomly oriented, tangled open spaces connected to each other and forming a network of liquid-wicking ducts. Porous materials may be formed from natural and/or synthetic sources, fibrous or particulate. Porous materials suitable for use herein are well known to those of skill in the art and include, for example and without limitation, nitrocellulose-based materials, polymer-based materials, acrylic-based materials, such as spun laced acrylic, and the like.

"Mobilizable," as used herein, refers to a reagent associated with a porous or non-porous material of a device in accordance with the present invention which reagent is stationary under one or more sets of conditions, and substantially movable under one or more different sets of conditions. Typically, reagents are stationary prior to, and become substantially mobilizable during, operation of a device of the present invention. By way of example, a mobilizable reagent may be a reagent that has been impregnated into a porous material or placed, for example dried, onto a non-porous component of a device of the present invention, such that during operation of the device, the reagent becomes substantially solubilized, hydrated or otherwise released from the porous or non-porous material, when contacted by the sample, and is carried laterally along with the sample as the sample progresses through the device. Preferably, the mobilized reagent and sample flow through the device at substantially equal rates and with relatively unimpaired flow. In another example, the mobilizable reagent is not dissolved, or solubilized, but is nevertheless substantially mobilized, i.e. released, and transported by the sample.

A "non-porous" material or component refers to a material through which insubstantial quantities of fluid (for example, liquid sample) flow. As used herein, typically fluid flows on or along a non-porous material or within an area (or channel or chamber) formed by multiple pieces of non-porous material. A non-porous flow path then refers to a flow path formed by one or more non-porous materials along which fluid flows. A porous material may be in fluid communication with a non-porous flow path. Such porous material may facilitate flow within the device by, for example, absorbing fluid from the non-porous flow path into or onto the porous region. Alternatively or additionally, a non-porous flow path may be constructed to form a capillary space through which fluid can flow by capillarity. Other alternative flow mechanisms will be apparent to those of skill in the art and are likewise contemplated herein. By way of example, fluidics, hydrostatic pressure and/or gravity may be exploited to facilitate fluid flow.

### II. Introduction

The present invention provides improved analytical devices and methods employing such devices for use in determining the presence or approximate quantity of one or more analytes in a liquid (i.e., fluid) sample. In particular, devices of the present invention comprise at least one secondary flow path which secondary flow path joins a primary flow path at a single junction. Secondary flow paths in accordance with the present invention are configured such that liquid sample present in the primary flow path enters the secondary flow path through the single junction and then subsequently is withdrawn back to the primary flow path, through the same single junction, as the liquid in the primary flow path is depleted (i.e., flows into the end of the assay). Withdrawal of liquid sample from the secondary flow path may be accomplished, for example, by absorption of liquid by a porous or absorbent material in fluid communication with the secondary flow path and/or by capillary action within the secondary flow path. In some embodiments provided herein, the secondary flow path is comprised of non-porous material having one or more reagents located thereon, which reagents are delivered sequentially into the primary flow path. A preferred reagent for subsequent delivery in accordance with the devices contemplated herein is one or more components of an enzyme-based label system, such as for example, an enzyme substrate. The reagent, for example, enzyme substrate is initially deposited in the secondary flow path and subsequently substantially mobilized by the sample. The secondary flow path also can be employed to collect liquid that acts as a wash following initial flow of liquid through the device. The secondary flow path, thus, allows delayed delivery of liquid alone or liquid with reagents (for example, tagging or labeling reagents, conditioning reagents and/or control reagents) to downstream elements. For example, delayed delivery of an enzyme substrate(s) from a secondary flow path into a primary flow path and to a capture zone comprising captured analyte tagged with an antibody-enzyme complex is useful to prevent premature delivery to, or mixing of, the enzyme substrate with the enzyme in the tag that could otherwise create a background color and prevent accurate interpretation of the assay result.

Among other advantages, the devices and methods of the present invention provide true single step assay formats that may employ any of various labeling systems including without limitation enzyme-based labels, colored moieties, fluorescent labels, etc.

### III. General Layout Of Device

In general, devices of the present invention may employ both porous and non-porous materials in the flow paths. In preferred devices, the secondary flow path is comprised of non-porous material and the capture zone, located within the primary flow path, is located on or within a porous material. To the degree an analytical device in accordance herewith relies on a directly visually detectable signal (label system) for detection of results, the capture zone is preferably located on/in a porous material in order to provide a higher concentration of capture reagent at the capture zone thereby permitting capture of a higher concentration of analyte and greater signal generation. Where a machine readable signal (for example, radioactivity, fluorescence or magnetism) is employed the capture zone may more readily be located on a non-porous material.

Devices of the present invention typically comprise a sample application site, sometimes referred to herein as a sample entry port, a tagging zone, a capture zone, a secondary flow path (typically comprising one or more reagents therein) and optionally a control zone, an end-of-assay zone, and/or an absorbent material, for example to facilitate flow of fluid from the upstream portion of the device to the downstream portion of the device. In preferred embodiments described herein, the device is designed to tag, capture and label one or more analytes in a fluid (liquid sample), thereby detecting the presence of, and optionally quantifying, the analyte(s). Alternatively, the devices may be used to tag and label one or more analytes in a fluid which tagged/labeled analyte is then captured and the presence or approximate quantity of the analyte determined. The use of a non-porous structure in at least a portion of the device enables the rapid delivery of sample to the capture zone and eliminates flow interference from porous material.

In further embodiments contemplated herein, one or more conditioning zones comprising conditioning reagents are present in the device. See, e.g., Figures 6 and 8. Conditioning reagents may improve the performance of the assay, including, e.g., changing the pH, salt concentration or metal ion concentration, adding or removing inorganic or organic components or detergents, blocking non-specific interactions or removal/filtering of large and/or interfering components of the sample, such as for example, red blood cells from whole blood samples. Conditioning zone(s) may be included at or near the point of sample application and/or in a location wholly within the primary or secondary flow path and/or on/in either or both porous or non-porous material.

In additional embodiments of the present devices, control reagents are likewise comprised within the device. As will be appreciated by those of skill in the art, such control reagents may be located in either the primary or secondary flow path, depending for example upon what type of control is used. If the control reagent is intended to simply indicate that sample has flowed into and out of the secondary flow path, then the control reagent is preferably located in the secondary flow path and more preferably at the same location or upstream of any other reagents in that flow path.

Broadly, the devices of the present invention are preferably laid out such that sample applied to the device flows into the primary flow path optionally contacting a conditioning zone. A portion of the sample then flows from the primary flow path into the secondary flow path. Within the secondary flow path the sample typically contacts a label reagent and may additionally or alternatively contact a conditioning zone. By virtue of the fluidics within the devices of the present invention, sample that flows into the secondary flow path dwells in that flow path until the sample in the primary flow path begins to evacuate the primary flow path. In the primary flow path the initial sample portion flowing therethrough preferably contacts a tagging zone, mobilizing the tag located therein, and may optionally contact one or more conditioning zones. The sample, now comprising tagged analyte if analyte is present therein, now flows into contact with the capture zone where tagged analyte, if present, is captured. The sample continues to flow past the capture zone and, optionally, into contact with one or more of a control zone, additional conditioning zone(s), an end-of-assay zone and then to the end of the assay, preferably into an absorbent material that, in part, acts as sink for the sample.

As liquid sample flows through the primary flow path, sample from the secondary flow path begins to enter the primary flow path, preferably carrying with it label mobilized from the secondary flow path. The sample from the secondary flow path flows downstream contacting the tagging zone, which preferably no longer comprises tag, and then contacting the capture zone, where label flowing with the sample is captured or otherwise reacts with the reagents captured at the capture zone, and a signal is generated. Sample continues to flow through the device contacting any remaining zones through to the end of the assay. Thus, preferably the bulk of liquid sample flows through the device to the end of the assay.

In some embodiments of the present invention, the primary flow path is in fluid communication with a tagging zone comprising a tag that is capable of binding to the analyte or a derivative thereof to form a complex. As sample contacts the tagging zone, the tag is released from the tagging zone, for example by hydration or solubilization, becoming mobile with the sample. As sample and tag mix, they combine to form tagged analyte. This reaction or association of tag and analyte (or derivative thereof) may occur in the tagging zone or downstream thereof. Preferably, all mobilizable tag present in the tagging zone is mobilized by the primary flow path sample prior to sample from the secondary flow path coming into contact with that zone. This is preferred where the secondary flow path comprises a component of the label system that must react with a component of the tag in order to generate signal. Tags include, affinity agents, such as, for example, antigens, haptens, antibodies, ligands, receptors, nucleic acid molecules, or chemical reactants. Tags may be linked to a directly detectable label, including but not limited to, a light absorbing particle such as colloidal gold/selenium or a colored latex particle, a light absorbing moiety, a phosphorescent moiety or particle, a fluorescent moiety or particle or a chemiluminescent moiety or particle, or the tag may be linked to an indirectly detectable label, such as an enzyme, for example including, without limitation, a hydrolase, esterase (for example, alkaline phosphatase) or oxidoreductase (for example, horseradish peroxidase). The tagging zone may be in a porous or non-porous portion of the primary flow path. In some embodiments, the tagging zone is in a non-porous portion of the device, downstream of the secondary flow path junction.

The tagged analyte, as well as any free tag or analyte or derivative thereof, then flow downstream along with the sample as it progresses through the device. Preferably, the liquid sample and its components flow at substantially equal rates through the device. Without being bound by any particular theory, the flow through the porous portion of the device is preferably non-bibulous, that is, interactions between the porous material itself and the sample components are negligible or minimal (interactions between the sample components and reagents on or in the porous material are, of course, expected and are not considered indicative of bibulous flow). The capture zone comprises a reagent immobilized on or in the device. In some embodiments, the capture zone is localized to a porous region of the device. The device components may be arranged such that a downstream portion of at least one non-porous flow path ends at a porous component such that when liquid flows to the end of the non-porous flow path, the liquid is absorbed into the porous component and continues downstream through the porous portion of the flow path. When the sample contacts the capture zone, the capture reagent binds and immobilizes the analyte, derivative thereof, and/or the tagged analyte (complex).

Depending upon the labeling system, the tag may comprise a directly detectable agent (e.g., colored moiety) or an indirectly detectable agent (including agents which comprise a linking member for linking to a another detectable agent). One example of an indirectly detectable agent is an enzyme that is detected when contacted with an enzyme substrate (e.g., in the case of an enzyme labeling system). In some embodiments, it is desirable to delay delivery of the enzyme substrate to the capture zone until after the initial flow of analyte and tag to the capture zone. Thus, in such embodiments, an enzyme substrate is preferably placed in the secondary flow path to allow for contact of the enzyme substrate with the tag after it is immobilized in the capture zone. An exemplary indirectly detectable agent comprising a linking member is an agent comprising biotin or (strept)avidin. Such an agent is able to "link" to, i.e., bind or otherwise associate with, a second linking member, in this example, either (strept)avidin or biotin, respectively, thereby associating with the analyte whatever molecule (for example, a colored moiety or enzyme) is bound to said second linking member.

The binding reagent located in the capture zone is capable of binding or otherwise interacting with the analyte or a component of the analyte complex to retain (immobilize) the analyte or component. For example in a sandwich assay format, the capture zone may contain an immobilized antibody that is a first binding partner of the target analyte and the mobilizable tag may contain an antibody that is a second binding partner of the analyte. Thus, at the capture zone, the analyte is sandwiched between the two antibodies.

In a competitive assay system, the analyte displaces or competes with its analog or derivative in the capture zone, wherein the analyte or its derivative are labeled in the tagging zone upstream of the capture zone.

In some embodiments, the capture zone is localized in a porous region of the device. The device components may be arranged such that a downstream portion of at least one primary non-porous flow path ends at a porous component such that when liquid flows to the end of the non-porous flow path, the liquid is absorbed into the porous component and continues downstream through the porous portion of the flow path.

In some embodiments, the device is designed such that the enzyme substrate does not contact or has minimal contact with free, mobile tag comprising enzyme label, which would otherwise cause background and/or false positive readings in the capture zone. In these embodiments, enzyme substrate arrives to the capture zone after separation of the free from capture-bound tag is largely accomplished, i.e., after the labeled analyte is bound to the capture zone. The result is a detectable, usually visual signal at the capture zone with little or no background. This may be, and preferably is, achieved by placing the enzyme substrate in the secondary flow path located upstream from the tagging zone wherein the secondary flow path is in fluid communication with the primary flow path.

In some embodiments, devices of the present invention are enclosed in a structure or housing. Such devices preferably further comprise a means for removing any air within the device that is displaced upon the introduction of fluid thereto, which means preferably also operate to permit re-entry of air into the device as fluid flows out of the flow paths and into the end of the assay. This will minimize the build-up of back pressure and or formation of a vacuum within the device that can prevent liquid from flowing through the device. For example, one or more air vents may be located in the device, such as at or near the upstream end of the secondary flow path (away from the junction of that flow path with the primary flow path) and/or at or near the end of the assay, in order to facilitate the movement of fluid into and out of the secondary flow path and through the primary flow path. Any means for affecting the airflow in the device is envisioned, including without limitation, vents, valves or vacuums. For example, the secondary flow path maybe designed both to facilitate fluid flow and to accommodate the displaced air within the device.

Additional embodiments may comprise, an air vent located at the upstream end of the secondary flow path, allowing for entry of fluid from the primary flow path and subsequent withdrawal of the fluid back into primary flow path when there is minimal or no sample in the sample entry port to support continued flow in the primary flow path. To trigger flow in the secondary flow path, the volume of the sample deposited in the primary flow path may be controlled. It will be appreciated by those of skill in the art that the primary flow path need not empty completely before fluid from the secondary flow path re-enters the primary flow path. Rather, as fluid is emptied from the primary flow path, such as by being absorbed into the absorbent material at the downstream end of the primary flow path, fluid from the secondary flow path is re-entering the primary flow path in an unremitting manner. Significantly, the design of the devices of the present invention provide a true one-step enzyme immunoassay.

In some embodiments, the sample port is in fluid communication with a chamber that is at a level below the level of the tagging zone. In these embodiments, the lower chamber is in fluid communication with the primary flow path such that the sample enters the flow path from the bottom of the flow path. See, e.g., Figures 3 and 5. In some embodiments, the sample enters the flow path from the bottom at a location between the location of the enzyme substrate and the tagging zone. In these embodiments, the sample enters the device, flows through the chamber and then enters the primary flow path at which point a first portion of the sample contacts the tagging zone without contacting the enzyme substrate while a second portion of the sample contacts the enzyme substrate (e.g., in a secondary flow path) and subsequently reverses direction and flows into the primary flow path following the initial sample portion. It will be appreciated by those of skill in the art that sufficient sample must be present in the lower chamber and sufficient flow rate must be achieved within the device such that sample flowing out of the secondary flow path flows across the junction of the lower chamber with the primary and secondary flow paths and into the primary flow path rather than back into the lower chamber.

Such devices of the invention employing a lower chamber optionally may include a conditioning zone within the lower chamber that, for example, binds components of a sample that directly or indirectly interfere with analyte detection. See, e.g., U.S. Patent No. 6,737,278. By binding the interfering components before they reach the capture zone, the conditioning zone may improve detection of the analyte or derivative thereof.

Devices of the present invention optionally may, and typically do, further include a control zone, preferably located near the capture zone. See, e.g., Figures 2-5. Particularly where the device is designed for detection of a single analyte in a liquid sample, the control zone is preferably located downstream of the capture zone. Where more than one analyte is to be detected and thus more than one capture zone is employed within the device, it may be preferable to locate a control zone between two capture zones, for example as a means for distinguishing one capture zoned form another. In one aspect, the control zone may be designed to generate a signal that indicates that the liquid sample has indeed flowed through the device past the capture zone, and therefore, that the assay is working as designed. The control zone generally will comprise an immobilized reagent ("immobilized control reagent") that either is capable of generating a detectable signal as a result of interaction with a component of the sample or, more preferably, is capable of binding a control reagent comprising a detectable moiety. By way of example, the control zone may comprise an antibody capable of binding (immobilizing) a component of the tag or capable of binding a separate control reagent comprising an antigen coupled to a colored moiety. Where a separate control reagent is used, such reagent is preferably located in the secondary flow path, such that immoblization of such control reagent in the control zone is indicative of sample flow from the secondary flow path through the device to the control zone. The component immobilized at the control zone may be directly or indirectly visualized. Optionally, the control zone may function as a "reference" zone to aid in determination of the presence of approximate quantity of the analyte in the aqueous sample or both control and reference zones may be employed in the device. Those of skill in the art will be familiar with such reference zones and readily able to implement such in the present devices.

Those of skill in the art will recognize that a control zone may also function as a negative or positive control. By way of example, a negative control zone may be designed to indicate non-specific or background levels of detectable label. This may be accomplished by preparing the negative control zone in the same manner as the capture zone but without the presence of the capture component of the capture reagent (such as the capture antibody). An exemplary positive control zone may be prepared by immobilizing authentic target analyte within the zone, which analyte will capture and immobilize tag and/or label reagents as a positive indication of operability of the assay.

The device may optionally include an end-of-assay zone that will indicate, for example, that sufficient sample has flowed through the device and/or that sample and reagents have reacted in the device for a sufficient amount of time to permit accurate interpretation of the assay results. As with other reagents employed in the devices of the present invention, end-of-assay reagents may be located within either or both of primary or secondary flow paths. By way of example, an end-of-assay reagent may be located in a secondary flow path such that detection of such end-of-assay reagent in the end-of-assay zone will evidence successful flow of sample from the secondary flow path through the device. An exemplary end-of-assay zone may comprise an immobilized binding reagent, such as an antibody, and an exemplary end-of-assay reagent may comprise a colored latex bead coupled to antigen specific for such antibody.

Further, embodiments of devices in accordance herewith may comprise a region at the downstream end of the primary flow path that may serve to take up the liquid sample and any unbound reagents. The end region may be an extension of a porous primary flow path or a different porous, absorbent material in fluid communication with the primary flow path, or a non-porous reservoir. Porous/absorbent material is preferred as such can facilitate flow through the device.

In some embodiments, the devices of the invention may be designed to detect and/or estimate the quantity of two or more analytes. In such embodiments, different tags, for example, different antibodies, as well as different labels may be provided for each analyte or derivative thereof. Moreover, the devices may comprise two or more capture zones in which a different analyte or derivative thereof is captured. Further, one or more control zones may be employed for example, to provide reference marks in order to distinguish different capture zones and hence, detection of different analytes.

Primary flow paths may comprise either porous or non-porous materials, or may be comprised of both porous materials and non-porous materials. In some embodiments, the devices comprise a primary flow path comprising a non-porous (optionally microfluidic) upstream region (e.g., comprising the tagging zone associated with the non-porous region) and a porous downstream region (e.g., comprising the capture zone associated with the porous region), wherein the zones are in fluid communication. For example, Figures 2A-B and 3A-C illustrate embodiments in which the tagging zone is associated with upstream portions that are non-porous and the capture zone is associated with downstream regions that are porous. In other embodiments the tagging zone may reside in the porous portion of the device, upstream from the capture zone, as illustrated on Figures 4 and 5.

Although the devices of the present invention are preferably employed as one-step assay devices, i.e. the assay only requires addition of the sample to the device, other assays utilizing the devices of this invention are likewise contemplated, for example other liquids, such as washes or conditioners, can be introduced into the device prior or subsequent to sample addition. Similarly, additional reagents and/or reactants may be added to the device prior or subsequent to addition of sample, including for example reagents to enhance signal generation indicating the presence or approximate quantity of analyte in a liquid sample.

### IV. Secondary Flow Paths

Secondary flow path(s) may include a porous portion as well, but are preferably non-porous. The secondary flow path can form a junction with any non-porous portion of the primary flow path. Preferably, a secondary flow path forms a junction with the primary flow path at a location where it is desired that at least some of the sample flow in the secondary flow path is delayed before reaching an area downstream of the junction in the primary flow path. In this case, a portion of the sample enters the secondary flow path while the remaining portion of the sample continues down the primary flow path(s). Once most or all of the sample in the sample entry port is drawn into primary flow path, then the flow continues by drawing the sample residing in the secondary flow path back into the primary flow path. Thus, in some embodiments, a secondary flow path forms a junction between the sample entry and the tag zone such that at least part of the sample is delayed in the secondary flow path before arriving at the tag zone.

In any event, the volume of the secondary flow path is such that it is sufficient to reach the capture zone and the optional control zone. In particular, the void volume of the porous primary flow path from the upstream edge to the control zone (or capture zone if no control zone is present) must be less than the volume capacity of the secondary flow path.

Figures 2B, 3C, 4B, and 5C exemplify the initial flow (solid line) of sample as a portion of the sample moves initially towards the tagging zone while another portion moves towards the enzyme substrate. The dashed lines in Figures 2B, 3C, 4B, and 5C illustrate the subsequent flow of the sample following exhaustion of the sample in the sample entry port. This later flow of sample is subsequently drawn towards and through the tagging and capture zones (such as, for example by absorption of the sample by the porous and/or absorptive components and/or by capillary action or laminar flow within the non-porous component). Liquid enters the secondary flow path from the primary flow path before it again exits the secondary flow path and re-enters the primary flow path.

In enzyme-based detection, premature delivery to, or mixing of, the enzyme substrate with the enzyme-labeled tag upstream from the capture zone can create an undesirable background that prevents accurate reading at the capture zone. Accordingly, it is desirable to deliver enzyme substrate after the majority of the free enzyme-labeled tag has flowed downstream to the capture zone. In many embodiments, therefore, it is useful to place the enzyme substrate in a non-porous secondary flow path that forms a junction upstream of the tagging zone. When the enzyme substrate is in the secondary flow path, the fluid that enters the secondary flow path substantially mobilizes, for example by solubilizing, the enzyme substrate and is subsequently drawn back into the primary flow path after most of the sample from the primary flow path has moved through with the enzyme.

In the devices of the present invention it is preferred that the only source of fluid to the secondary flow path come from the primary flow path (including the sample entry port or path). Thus, liquid only enters the secondary flow path from the primary flow path before it again exits the secondary flow path and re-enters the primary flow path. Thus, the secondary flow paths of the present invention typically do not comprise any reservoirs containing fluid prior to entry of the sample from the primary flow path into the secondary flow path nor do the secondary flow paths comprise an entry port for addition of a second fluid. In many embodiments, the secondary flow paths form only one junction with the primary flow path. However, as described below, in some cases the secondary flow path will be in fluid communication with two primary flow paths, thereby connecting the two primary flow paths. In some embodiments, the secondary flow path may contain stabilizers, buffering components and other reagents in addition to the enzyme substrate or other detection or labeling systems. In some embodiments, a tag is associated with (e.g. conjugated to) a first linking member (e.g. (strept)avidin). In these embodiments the secondary flow path may contain a second linking member, associated with a colored moiety that binds to the first linking member (e.g. biotin). This conformation allows for detection of the tag and therefore the analyte. Examples of linking member pairs include, e.g., biotin and (strept)avidin or fluorescein and anti-fluorescein.

### Placement of Secondary Flow Paths

As illustrated in Figure 1, secondary flow paths can be placed in many orientations with respect to the primary flow path and tagging zone. In some cases, the sample entry and tagging zone are in a substantially straight flow path, with a secondary flow path forming a junction with the primary flow path at a point between the sample and the tag. See, e.g., Figure 1A. Alternatively, Figure 1B illustrates an embodiment in which the primary flow path divides into two primary flow paths at an upstream junction and then merges at a junction downstream from the secondary flow path.

In yet further embodiments, the primary flow path may form an angle between its upstream end (sample entry port) and the tagging zone. As illustrated in Figures 1C-E, a secondary flow path may be placed so that it forms a substantially straight flow path with the tagging zone located in the primary flow path. This aspect is also depicted in, e.g., Figure 2B.

The flow capacity of any particular flow path (e.g., as represented by the cross-section of a porous or non-porous flow path) need not remain constant through its entire course.

Secondary flow paths will generally comprise means for removing air within the device that is displaced upon introduction of a liquid sample thereto. In some embodiments, one or more vents are employed for this purpose. By way of example, a vent may be located at the upstream end of the secondary flow path to permit the escape of air therefrom as the liquid sample flows into that flow path. Similarly, and by way of further example, air vents may be employed as well near the end of the assay to facilitate flow of sample through the device to that end. Air vents generally will be located in the top or along the side of a device, provided however that sample preferably does not escape the device through such vent(s). By way of example, Figure 8 illustrates an embodiment of the present invention wherein the air vents are configured such that air within the device flows up and to the side to escape the device. As illustrated, this is accomplished by forming a vent in the top of the top cover of the device, affixing spacer material to the top cover leaving the air vent (as well as sample entry port and read window) uncovered and then affixing a vent cover to the spacer material, which vent cover comprises cut-outs for the sample entry port and read window but not for the vents.

### V. Labeling Agents

Those of skill in the art will readily appreciate various labeling systems that may be employed in the devices of the present invention. Exemplary labels include without limitation, light absorbing particles such as colloidal gold/selenium or colored latex particles, phosphorescent moieties or particles, fluorescent moieties or particles, dyes (for example, polymerized dyes) or sols, or colored or fluorescent or chemiluminescent molecules or particles or colored insoluble products of an enzyme action and/or radioactive molecules. Preferably, the signal generated by the label is optically detectable, such that it may be detected through an optically clear or transparent read window or region and related to the presence and/or amount of analyte in the sample.

In one preferred signal-generating system, a soluble enzyme substrate is employed as a label which substrate lacks a substantial absorption in the visible spectrum but is converted into a water-insoluble and visible (i.e., with a substantial absorption in the visible wavelength region) product upon action of an enzyme which enzyme preferably has been incorporated into the tag. Exemplary enzymes include, without limitation, alkaline phosphatase, beta-galactosidase, horseradish peroxidase and penicillinase. Those of skill in the art are well aware of various substrates that may be used in such an enzyme-based labeling system. Choice of substrate will depend, for example, upon the enzyme employed, the sensitivity desired (for example will color generation alone be enough to visualize a result or is fluorescence preferred) and the like. Alternatively, an enzyme substrate, with or without a substantial absorption in the visible spectrum, which is converted into a fluorescent or luminescent product by an enzyme may be employed.

An example of some embodiments which comprise an analytical device wherein determination of the presence or approximate quantity of the analyte in the liquid sample comprises detection of an optically detectable signal are also contemplated in the present invention wherein: an analytical device comprising: a primary flow path; a capture zone, capable of immobilizing the analyte within the primary flow path, and a secondary flow path adjoining the primary flow path at a single junction upstream of the capture zone, wherein the only source of fluid to the secondary flow path is from the primary flow path. Further wherein the primary flow path may comprise porous and non-porous material and wherein the secondary flow path may comprise of non-porous material. An embodiment may further comprise a tagging zone within the primary flow path comprising at least one tag which is capable of forming a complex with the analyte, wherein said tag is substantially mobilizable when contacted with the liquid sample; and wherein the tagging zone may be located upstream of and may be in fluid communication with the capture zone; and wherein the capture zone may comprise an immobilized capture reagent which may be capable of binding the analyte. An embodiment may further comprise a label in the secondary flow path wherein said label may comprise a first linking member coupled to a colored moiety and said tag may comprise a second linking member and wherein said first linking member is capable of binding said second linking member. An analytical device may further include an embodiment wherein analyte present in the liquid sample binds said tag and is bound by said immobilized capture reagent; and wherein said first linking member coupled to said colored moiety is bound to said second linking member comprised within said tag, thereby providing an optically detectable signal within said capture zone. An analytical device may further include an embodiment wherein said first linking member and said second linking member are different and are biotin and avidin respectively or avidin and biotin respectively.

### VI. Construction Of Device

Devices in accordance with the present invention may be constructed by any of a variety of means well known by those of skill in the art. In some embodiments, the body structure of a non-porous portion of the device of the present invention consists of an assemblage of multiple (e.g., three or more) separate layers which, when appropriately joined together, form a non-porous flow path comprised within the device. See, e.g., Figures 2A and 3A. As shown in these figures, such a non-porous structure may consist of a top cover, a bottom support, and an interior portion or "cut-out", wherein the cut-out substantially defines the flow paths of the device. A thin film (e.g., a Mylar^{®} polyester film) may be applied as the top cover and/or bottom support to seal the void created by the cut-out layer.

Any means may be used to mate a porous material of the device with a non-porous material and/or to secure non-porous material to one another, provided however; such means do not interfere with the flow dynamics within the device. By way of example, the interior (cut-out) portion of the non-porous structure of the device of the present invention may be constructed of a double-sided adhesive in which the flow paths (channel(s)/chamber(s)) are created by die cutting. After removal of the release liners, the cut-out portion of the non-porous structure is then mated to, e.g., placed into contact with, and bonded to the planar surface of, the bottom support. Such may be accomplished in a continuous manufacturing process or manually. Alternatively, the shape of the non-porous flow path may be formed by embossing, molding, etching, photolithography or laser ablation.

Manufacturing methods may comprise depositing at least one biological reagent at one or more predetermined positions on an assay support using, for example, a flexographic process. An introduction to flexography is found in, e.g., Encyclopedia of Chemical Technology (Kirk-Othmer, eds., 1993), vol. 20, pp. 101-05, and the references cited therein. The methods may generally include combining various support and matrix (such as porous materials) layers into a laminate device, application of reagents including the application of biological reagents using flexography, cutting the devices from a web with optional housing and final pouching of product. The predetermined positions of the deposited reagents are generally within a flow path of the device and the biological or binding reagent generally retains a substantial native biological activity. In some aspects of the method, the flexographic process includes an anilox roller system in operable relationship to a printing plate and a reagent vessel containing the biological reagent such that the biologically active reagent is repetitively printed on the assay support. The assay support used may comprise a porous or non-porous, water impermeable material in a continuous web form. In some embodiments, continuous process(es) are carried out in a web format where tagging reagent(s), labeling reagent(s), such as enzyme substrate(s) if necessary, or other chemical compositions (such as e.g., salts, polymers, agglutinins or buffering formulations) for sample treatment are deposited in the desired locations within the appropriate channels or flow paths.

The top cover and bottom support of the non-porous structure preferably consist of a solid, planar material. The top portion may incorporate holes (vents) that may be fabricated using, for example, die cutting carried out in a continuous process in a web format. The holes in the top portion of the non-porous structure are preferably oriented such that they are in communication with the flow paths (channels and/or chambers) formed in the interior portion of the structure. In the completed device, these holes may function, e.g., as inlet ports for introduction of sample into the interior of the device or as vents in the device to facilitate fluid flow therein, for example, a vent may be located over the flow path comprising the labeling element(s) (such as, enzyme substrate(s)) and/or may be located at the downstream end of the device, for example adjacent an absorbent material located at the end of the assay and/or a vent may be located adjacent the capture zone and/or control zone thereby serving both as a vent and as read window. The positions and/or size of these holes as well as the geometry of the flow paths may be varied as needed to control the desired sequential fluid flow within the device thereby providing the desired sequential reagent delivery.

One or more porous components may be mated to the non-porous structure by being sandwiched between the top cover and bottom support portions of the non-porous component of the device, so long as fluid flow proceeds through the porous material rather than over, under or otherwise around it. The top cover portion of the non-porous structure may be bonded with the planar top surface of the cut-out portion, thereby covering and sealing the cut-out portion to form the flow paths (channels and/or chambers) of the device enclosed between the top cover and bottom support components.

Some reagents, for example conditioning reagents, may alternatively be deposited on the bottom surface of the top cover portion. Printing the same reagent on both the top and bottom non-porous portions of a flow path also can be used to increase the amount of the reagent available for the assay.

In some embodiments, the non-porous structure will include on its top surface an opaque covering layer (which may, for example, contain artwork) that may merely be introduced in a continuous, flexographic process of either printing of desired ink(s) or lamination of non-transparent polymer material(s) or may be manually affixed to the device.

A variety of materials may be employed as the bottom support or top cover portion of the non-porous structure. When the devices of the present invention are manufactured in continuous processes carried out in a web format, materials are preferably selected based upon their compatibility with known converting techniques, e.g., die cutting, printing, lamination, embossing, island placing, and other techniques. The materials are also generally selected for their compatibility with the full range of conditions to which the devices of the present invention may be exposed, including extremes of pH, temperature, salt concentration, and that are inherently compatible without undesired interference by components within a bodily fluid sample containing the analyte(s) of interest such as whole blood, whole blood-derived specimens (e.g., plasma or serum), urine, saliva, sweat, fecal, vaginal, and sperm samples, and specially treated samples (for example, extracted samples for infectious disease testing).

In some embodiments, the structural materials will consist of polymeric materials, e.g., plastics, such as polyesters, polyethlylene, polymethylmethacrylate (PMMA), polycarbonate, polyvinylchloride (PVC), polysulfone, polivinylidene fluoride (PVDF) and the like. These polymeric materials may include treated surfaces, e.g., derivatized or coated surfaces and or other physico-chemical alterations, to enhance their utility in the devices of the present invention, e.g., by providing enhanced fluid flow or improving printing compatibility.

Preferably the non-porous material is a polyester film, such as those made from polyethylene terephthalate and known as Mylar^{®}.

Porous materials or components can be formed by processing natural fibrous components, such as, for example, cellulose derivatives, and presented in a suitable finished form of a paper-like material or synthetic components, such as, for example, glass fiber or the mixture thereof blended with synthetic binders (such as, for example, polymeric alcohols and/or vinyl derivatives) and presented in a suitable finished form of a filter-like material.

Alternatively, porous components are formed from non-woven and non-fibrous materials, polymers selected from, for example, cellulose and its derivatives (e.g., nitrocellulose, cellulose esters and regenerated cellulose), poly-alkylenes (e.g., polyethylene), halogen-substituted poly-carbons (e.g., PVDF, and the like), nylon 66 derivatives.

Preferably the porous material comprises a nitrocellulose membrane (Millipore Corporation, Bedford, MA) or polyethylene membrane, such as POREX^{®} Lateral-Flo™ membrane (Porex Technologies Corporation, Fairburn, GA) or other sintered polymer membranes described, for example, in published US Patent Application Number 2003/0096424, published May 22, 2003.

### VII. Uses

The analyte may be any compound that can be detected, e.g. small organic molecules, peptides and proteins, sugars, nucleic acids, complex carbohydrates, viruses, bacteria particles (bacteria, bacterial extracts), lipids and combinations thereof, naturally occurring or synthetic or combinations thereof. The analytes may include, but are not limited to, drugs, both naturally-occurring and synthetic, various components of animals, including humans, such as blood components, tissue components, and the like; microorganisms, such as bacteria, fungi, protozoa, viruses, and the like; components of waste or products or contaminants of such products in commercial processing; components of the environment, particularly contaminants, such as pesticides, microorganisms, and the like.

In carrying out an assay in the device, one may assay any type of liquid, provided however, such liquid flows naturally or may be treated to be able to flow properly within the device. By way of example, a particularly viscous liquid may need to be diluted prior to use in the assay or such sample may need to have analyte extracted therefrom into solution and the extracted sample applied to the device. The liquid sample may be a contrived (spiked) sample or may be a natural sample from any source, such as a physiological source, e.g. blood, serum, plasma, urine, saliva, spinal fluid, lysate, nasal pharyngeal aspirates etc.; sample of ecological interest, e.g. water, soil, waste streams, organisms, etc.; food, e.g. meat, dairy products, plant products, other organic matter etc.; drugs or drug contaminants in processing; or the like. Depending upon the nature of the sample, the sample may be subjected to prior treatment, such as extraction, distillation, chromatography, gel electrophoresis, dialysis, dissolution, centrifugation, filtration, cell separation, and the like. For blood, one may wish to remove red blood cells and use plasma or serum.

### VI. Housing

The devices of the invention may be contained or enclosed in a housing. The housing can be of any design or construction (e.g., molded, embossed or laminated plastic) so long as it does not interfere with the flow of the sample within the flow paths and allows for reading of the sample when an assay is complete. The housing may comprise any or all of the following:
(1) a sample entry port for receiving a liquid sample;
(2) a reading access for permitting the capture zone to be read, thus allowing determination of a presence or absence or approximate quantity of an analyte in a sample by reading the capture zone; and, optionally,
(3) a reading access for permitting a control zone to be read,
(4) a reading access for permitting an end-of-assay zone to be read, and/or
(5) one or more air vents to facilitate sample flow.

Having now generally described the invention, the following examples are provided for illustration and are not intended to be limiting of the present invention.

### EXAMPLES

### Example 1 - Preparation of Alkaline Phosphatase hCG Antibody Conjugate Tag

Common chemicals were either of analytical reagent grade or highest purity commercially available. A goat antibody against hCG (human chorionic gonadotropin) was conjugated to a calf intestinal alkaline phosphatase using heterobifunctional thioether chemistry as follows.

A recombinant alkaline phosphatase (Roche Diagnostics GmbH, Mannheim, Germany) was diluted with 0.1 M sodium phosphate buffer (pH 7.5) and a limited number of amino groups of the enzyme were substituted with maleimides using sulfo-SMCC (Pierce Chemical Company, Rockford, IL) by incubation at room temperature. The modified enzyme was purified by a gel filtration on a Sephadex G-25 column (Amersham Biosciences Corporation, Piscataway, NJ) equilibrated in 0.1 M sodium phosphate (pH 7.5) and a number of maleimide groups introduced were determined. AF(ab')₂ fragment of an anti-hCG polyclonal goat antibody (Quidel Corporation, San Diego, CA) in 50 mM Tris-0.5M NaCl-0.1% sodium azide buffer (pH 8.0) was reacted with 2-mercaptoethylamine hydrochloride (TCI America, Portland, OR) at 37°C under a nitrogen atmosphere.

The reduced antibody was purified by a gel filtration on a Sephadex G-25 column and molar equivalents of thiols introduced into the antibody were determined. A conjugation reaction between the modified alkaline phosphatase and antibody was carried at room temperature in 0.1 M sodium phosphate buffer (pH 7.5) at room temperature.

After quenching of the unreacted maleimides and thiols with 2-mercaptoethanol and N-ethylmaleimide, respectively, the conjugate was fractionated by a gel filtration on a Sephacryl S-300 HR column (Amersham Biosciences Corporation) equilibrated with 50 mM Tris HCl, buffer (pH 8.0) containing 1mM MgCl₂, 0.1 mM ZnCl₂,1 mg/ml of bovine serum albumin (BSA; Biocell Laboratories Inc., Rancho Dominguez, CA) and 0.1 % sodium azide. The resultant conjugate was assessed for its efficiency to detect the hCG in the analytical devices described in the subsequent examples.

### Example 2 - Preparation Of A Porous Flow Path

A 2.5-cm wide strip of a 9 mm thick microporous polyethylene membrane ("Porex^{®} membrane"; Porex Technologies Corporation; see US Published Patent Application 2003/0096424, especially Example 1) was laminated with a 2 mm thick double-coated adhesive tape (Adhesive Research Inc., Glen Rock, PA) by rolling through a pressure roller. After 24-hr curing period necessary to establish bonding between the two layers of the laminate, the analyte-specific and control capture lines of 0.5 to 2.0 mm width were striped using a X-Y Plotter equipped with a Rapidograph pen (1 mm dispensing tip; Koh-I-Noor Inc., Leeds, MA) along the length of the strip.

A monoclonal antibody against beta subunit of hCG (Scripps Laboratories Inc., San Diego, CA) prepared in 100 mM POPSO buffer (pH 7.5) containing 250 mM NaCl was striped at a 7 mm distance from an edge of the Porex membrane. A goat antibody against alkaline phosphatase (Biodesign International, Saco, ME) prepared in 25 mM Tris-citrate buffer (pH 5.0) was striped at 11 mm distance from an edge of the membrane.

A conditioning solution for a sample suspected of containing an analyte comprised BSA dissolved in 250 mM POPSO buffer (pH 7.5) containing Triton X-100 (Sigma Chemical Company, St. Louis, MO). The conditioning solution was striped at a 1 mm distance from the edge of the membrane using the Rapidograph pen comprising a 2 mm dispensing tip. The resultant laminate strips were dried for 10 min at 45°C in a convection oven and subsequently stored in a nitrogen-flushed box.

### Example 3 - Preparation Of The Non-Porous Flow Path

As shown in Fig. 6 (the "Cut-out"), a shape of a non-porous flow path was cut-out in the 10 mm thick double-coated adhesive tape (Lohmann Technologies, Hebron, KY) using rotary die cut followed by lamination to the clear 7 mm clear bottom polyester Mylar^{®} tape (Transilwrap Company, Inc., Franklin Park, IL) in a continuous web process. The resultant web of non-porous flow paths was cut into 10.5 inch-long panels comprising 10 test devices each.

The alkaline phosphatase-antibody conjugate tag stock (see Example 1) was diluted with 50 mM Tris buffer (pH 8.0) supplemented with BSA, sucrose, poly(vinyl alcohol) (PVA), MgCl₂, ZnCl₂, calf intestinal alkaline phosphatase (Calzyme Laboratories Inc., San Luis Obispo, CA), and Proclin 300 (Supelco, Bellefonte, PA) as a preservative. A substrate for either the alkaline phosphatase or the conjugate of the alkaline phosphatase with the antibody was prepared in a formulation consisting of two parts, Part A and Part B.

Part A included a disodium salt of 3-indoxyl phosphate (3-IP; Biosynth International Inc., Naperville, IL), sorbitol, diethanolamine, OGME (octaethylene glycol monododecyl ether) and PVA dissolved in methanol. Part B comprised 2.5 M sodium carbonate L-tartaric acid buffer (pH 10.0). After mixing 48 parts by weight of Part A with 52 parts by weight of Part B, the substrate was applied to the device within 15 minutes.

The device panel was attached to the positioning template and solutions of the substrate and the conjugate were applied to the bottom clear polyester Mylar® portion of the devices as indicated in Fig. 2B. The substrate was positioned at least 1 mm upstream and to the right from the sample entry channel as a 5x5 mm square by spreading 1.5 µl of the solution with a micropipette tip. The conjugate was positioned at least 1 mm downstream and to the left from the sample entry channel as a 2x5 mm rectangle by spreading 1.5 µl of the solution with a micropipette tip. The resultant panel comprising the devices was dried at 45°C in a convection oven for 10 min.

### Example 4 - Completion Of The Device

The laminated strip, described in Example 2 and comprising the membrane with striped capture and control antibodies, was sliced to 8 mm wide test strips to fit the expanded portion of the non-porous flow path (9 mm, Fig 2A, middle layer and Fig 2B). After removal of a liner to expose an adhesive, the test strip was affixed to the expanded portion of the channel at no more then 2 mm downstream and to the left from the deposited conjugate (Fig 2B).

In order to construct a top cover, a strip of the 7 mm clear polyester Mylar tape was cut to cover both an edge of the porous capture strip with a 2 mm overlap and the entire substrate location (Fig. 6, the "Cover"). Subsequently, to complete a construction of the top cover, a sample port registering with the sample channel and a substrate vent that extended slightly beyond the substrate location were cut in the Mylar strip. Finally, a liner was removed from the adhesive in the non-porous flow path (the "Cut-out" on Fig 6) and the completed top cover was adhered to it. A 25 cm long and 20 mm wide strip of an absorbent paper (Ahlstrom Filtration Inc., Madisonville, TN) was placed over capture strips at a location 15 mm downstream from an edge of the Porex membrane and adhered to the adhesive of the cutout.

### Example 5 - Performance Of The Manually Assembled Devices

The test devices were prepared as described in Examples 2-4 by applying the antibody-enzyme conjugate tag diluted to 50, 100, 200 or 400 µg/ml. 75 µl of a female urine pool (hCG-negative sample) or the pool spiked with hCG (Quidel Corporation) to the level of 25 mIU/ml (hCG-positive sample) were added to the sample ports of the test devices. Devices were monitored for the appearance of an hCG-specific blue capture line.

The period of time to appearance of a faint color signal ("time to signal") from the positive samples was recorded. No visible signal was present in the devices tested with negative samples at all conjugate levels. Signal appeared progressively faster with increasing levels of the conjugate, while specificity of the assay remained unchanged.

### Example 6 - Fluorescence-based immunoassay for hCG

A water-insoluble undercoat ink for covering top and bottom surfaces of the nonporous flow path was prepared by dissolving poly(vinyl acetate) (Scientific Polymer Products Inc., Ontario, NY) in propyl acetate using an electrical mixer with a metal propeller paddle. A printable substrate for alkaline phosphatase was formulated as a propanol suspension as follows: sodium carbonate, mannitol, OGME, diethylamine, disodium salt of AttoPhos^{®} Substrate (disodium salt of 2'-[2-benzothiazoyl]-6'-hydroxybenzothiazole phosphate; Promega Corporation, Madison, WI) were combined and pulverized at room temperature with a high speed blender homogenizer. In the next step, fumed silica (Sigma Chemical Company) and polyvinylpyrrolidone (PVP) were dissolved in the suspension. The resultant ink was then ready for printing or refrigerated storage prior to printing. A sample conditioning ink was prepared by dissolving BSA and trehalose in 2 M Tris-HCl (pH 8.0) followed by an addition of Proclin 300 (Supelco, Bellefonte, PA) as a preservative. This ink too was then ready for immediate use or refrigerated storage.

Next, a tag printing ink was made in two steps. In the first step, 2 M Tris-HCl buffer (pH 8.0) was mixed with the tag stock and calf intestine alkaline phosphatase described in Example 3. In the next step, trehalose, MgCl₂, Blocking Peptide Fragment (Toyobo, Osaka, Japan) and bovine Poly-Pep (Sigma Chemical Company) were sequentially dissolved to yield the final tag printing ink. A porous flow path comprising capture and control lines were prepared using a continuous web process like that described in Example 2. Subsequently, the analytical devices for the fluorescence-based immunoassay for hCG were created in a manner and with the materials described in Example 4 by adding porous flow path, top cover and absorbent.

In operation, AttoPhos® Substrate (2'-[2-benzothiazoyl]-6'-hydroxybenzothiazole phosphate; Promega Corporation, Madison, WI) is cleaved by alkaline phosphatase to produce inorganic phosphate and the alcohol, 2'-[2-benzothiazoyl]-6'-hydroxybenzothiazole (BBT). This enzyme-catalyzed conversion of the phosphate form of AttoPhos® Substrate to BBT is accompanied by an enhancement in fluorescence properties. Relative to AttoPhos® Substrate, BBT has greatly increased quantum efficiency, and fluorescence excitation and emission spectra that are shifted well into the visible region. Relative to other fluorometric reporters, the BBT anion has an unusually large Stokes' shift of 120 nm, which leads to lower levels of background fluorescence and higher detection sensitivity. In order to assess performance of the fluorescence-based analytical devices, 75 µl of a female urine pool (hCG-negative samples) or the pool spiked with hCG (Quidel Corporation) to the level of 0.8, 6 or 25 mIU hCG/ml (hCG-positive samples) was added to each device. Devices were illuminated with a 400 nm light emitting diode and emission fluorescence was monitored at 500-600 nm using an uncooled charge coupled device. The results demonstrated an analytical sensitivity of 0.8 mIU hCG/ml at 10 min after addition of the sample to the device.

As used herein, the term "a", "an", and "any" are each intended to include both the singular and plural forms.

Having now fully described this invention, it will be appreciated by those skilled in the art that the same can be performed within a wide range of equivalent parameters, concentrations, and conditions without departing from the scope of the invention and without undue experimentation. While this invention has been described in connection with specific embodiments thereof, it will be understood that it is capable of further modifications. This application is intended to cover any variations, uses, or adaptations of the invention following, in general, the principles of the invention and including such departures from the present disclosure as come within known or customary practice within the art to which the invention pertains and as may be applied to the essential features hereinbefore set forth.

## Claims

1. An analytical device for performing an assay to determine the presence or approximate quantity of an analyte in a liquid sample, the device comprising:
a primary flow path;
a capture zone, capable of immobilizing the analyte within the primary flow path; and
a secondary flow path;
wherein the only source of fluid to the secondary flow path is from the primary flow path, and **characterised in that**:
the secondary flow path adjoins the primary flow path at a single junction only, upstream of the capture zone.

2. The device of Claim 1 wherein the primary flow path comprises porous and non-porous material.

3. The device of Claim 1 wherein the secondary flow path is comprised of non-porous material.

4. The device of Claim 1 further comprising:
a tagging zone within the primary flow path comprising at least one tag which is capable of forming a complex with the analyte,
wherein said tag is substantially mobilizable when contacted with the liquid sample;
wherein the tagging zone is located upstream of and in fluid communication with the capture zone; and
wherein the capture zone comprises an immobilized capture reagent capable of binding the analyte.

5. The device of Claim 4 wherein the tagging zone is located downstream of the junction of the secondary flow path with the primary flow path.

6. The device of Claim 4 wherein the primary flow path comprises porous and non-porous material and wherein the tagging zone is located on the non-porous material and the capture zone is located on or in the porous material.

7. The device of Claim 4 wherein the primary flow path comprises porous and non-porous material and wherein the tagging zone and capture zone are located on or in the porous material.

8. The device of Claim 1 further comprising an absorptive material downstream of, and in fluid communication with, the capture zone.

9. The device of Claim 4 wherein the tag comprises a visually detectable label selected from the group consisting of a colored moiety, a fluorescent moiety, a chemiluminescent moiety and a phosphorescent moiety.

10. The device of Claim 4 wherein the tag comprises an enzyme, which enzyme is capable of reacting with an enzyme substrate to generate a visually detectable signal.

11. The device of Claim 10 wherein the enzyme is selected from the group consisting of hydrolases, esterases and oxidoreductases.

12. The device of Claim 10 further comprising a label reagent located in the secondary flow path said label reagent comprising an enzyme substrate.

13. The device of claim 12, wherein a visually detectable signal is generated by reaction of the enzyme with the enzyme substrate.

14. The device of Claim 1 further comprising a control zone in the primary flow path.

15. The device of Claim 1 further comprising an end-of-assay zone in the primary flow path downstream of the capture zone.

16. The device of Claim 15 wherein the end-of-assay zone comprises a reagent that produces a detectable signal when contacted with the sample.

17. The device of Claim 1 further comprising a conditioning zone in the primary flow path.

18. The device of Claim 1 further comprising a housing.

19. The device of Claim 18 further comprising a means for displacing air from the device wherein said air is displaced upon introduction of liquid sample to the device.

20. The device of Claim 19 wherein the means for removing air comprises at least one air vent.

21. The device of Claim 20 comprising an air vent located at the upstream end of the secondary flow path.

22. The device of Claim 21 further comprising an air vent at the downstream end of the device.

23. The device of Claim 4 further comprising a label in the secondary flow path.

24. The device of Claim 23, wherein determination of the presence or approximate quantity of the analyte in the liquid sample comprises detection of an optically detectable signal.

25. The device of Claim 24, wherein said label comprises a first linking member coupled to a colored moiety and said tag comprises a second linking member and wherein said first linking member is capable of binding said second linking member.

26. The device of Claim 25, wherein analyte present in the liquid sample binds said tag and is bound by said immobilized capture reagent; and wherein said first linking member coupled to said colored moiety is bound to said second linking member comprised within said tag, thereby providing an optically detectable signal within said capture zone.

27. The device of Claim 26 wherein said first linking member and said second linking member are different and are biotin and avidin respectively or avidin and biotin respectively.

28. A method for determining the presence or approximate quantity of at least one analyte in a liquid sample, comprising:
applying the liquid sample to the primary flow path of the device of Claim 1; and
determining the presence or approximate quantity of the analyte in the capture zone.

29. The method of Claim 28, wherein the device further comprises a tagging zone located upstream of the capture zone and downstream of the junction of the secondary flow path with the primary flow path.

30. The method of Claim 28, wherein the primary flow path comprises porous and non-porous material.

31. The method of Claim 28, wherein the device further comprises an absorptive material downstream of, and in fluid communication with, the capture zone.

## Patentansprüche

1. Analysenvorrichtung für die Durchführung eines Assays zur Bestimmung des Vorhandenseins oder der angenäherten Menge eines Analyten in einer Flüssigprobe, wobei die Vorrichtung das Folgende umfasst:
einen primären Strömungsweg;
eine Einfangzone, die zur Immobilisierung des Analyten innerhalb des primären Strömungswegs fähig ist; und
einen sekundären Strömungsweg;
wobei die einzige Fluidquelle für den sekundären Strömungsweg von dem primären Strömungsweg stammt, und **dadurch gekennzeichnet, dass**:
der sekundäre Strömungsweg stromaufwärts von der Einfangzone an den primären Strömungsweg nur an einer einzigen Berührungs- bzw. Anschlusszone angrenzt.

2. Vorrichtung gemäß Anspruch 1, wobei der primäre Strömungsweg poröses und nichtporöses Material umfasst.

3. Vorrichtung gemäß Anspruch 1, wobei der sekundäre Strömungsweg aus nicht-porösem Material besteht.

4. Vorrichtung gemäß Anspruch 1, weiterhin umfassend:
eine Markierungs- bzw. Etikettierungszone innerhalb des primären Strömungswegs, umfassend mindestens eine Markierung, die zur Bildung eines Komplexes mit dem Analyt fähig ist,
wobei die Markierung im Wesentlichen mobilisierbar ist, wenn sie mit der Flüssigprobe in Kontakt gebracht wird;
wobei die Markierungszone stromaufwärts der Einfangzone und in Fluidverbindung damit angeordnet ist; und
wobei die Einfangzone ein immobilisiertes Einfangreagens umfasst, das zum Binden des Analyten in der Lage ist.

5. Vorrichtung gemäß Anspruch 4, wobei die Markierungszone stromabwärts der Berührungszone des sekundären Strömungswegs mit dem primären Strömungsweg angeordnet ist.

6. Vorrichtung gemäß Anspruch 4, wobei der primäre Strömungsweg poröses und nichtporöses Material umfasst und wobei die Markierungszone auf dem nicht-porösen Material angeordnet ist und die Einfangzone auf oder in dem porösen Material angeordnet ist.

7. Vorrichtung gemäß Anspruch 4, wobei der primäre Strömungsweg poröses und nichtporöses Material umfasst und wobei die Markierungszone und die Einfangzone auf oder in dem porösen Material angeordnet sind.

8. Vorrichtung gemäß Anspruch 1, weiterhin umfassend ein absorptionsfähiges Material stromabwärts der Einfangzone und in Fluidverbindung damit.

9. Vorrichtung gemäß Anspruch 4, wobei die Markierung eine optisch nachweisbare Markierung umfasst, gewählt aus der Gruppe bestehend aus einem gefärbten Komponententeil, einem fluoreszierenden Komponententeil, einem chemolumineszenten Komponententeil und einem phosphoreszierenden Komponententeil.

10. Vorrichtung gemäß Anspruch 4, wobei die Markierung ein Enzym umfasst, wobei das Enzym zum Reagieren mit dem Enzymsubstrat fähig ist zur Erzeugung eines optisch nachweisbaren Signals.

11. Vorrichtung gemäß Anspruch 10, wobei das Enzym aus der Gruppe bestehend aus Hydrolasen, Esterasen und Oxidoreduktasen gewählt wird.

12. Vorrichtung gemäß Anspruch 10, weiterhin umfassend ein Markierungsreagens, welches in dem sekundären Strömungsweg angeordnet ist, wobei das Markierungsreagens ein Enzymsubstrat umfasst.

13. Vorrichtung gemäß Anspruch 12, wobei ein optisch nachweisbares Signal durch die Reaktion des Enzyms mit dem Enzymsubstrat erzeugt wird.

14. Vorrichtung gemäß Anspruch 1, weiterhin umfassend eine Kontroll- bzw. Regulierungszone in dem primären Strömungsweg.

15. Vorrichtung gemäß Anspruch 1, weiterhin umfassend eine Assay-Ende-Zone in dem primären Strömungsweg stromabwärts der Einfangzone.

16. Vorrichtung gemäß Anspruch 15, wobei die Assay-Ende-Zone ein Reagens umfasst, welches ein nachweisbares Signal bei Kontakt mit der Probe erzeugt.

17. Vorrichtung gemäß Anspruch 1, weiterhin umfassend eine Konditionierungszone in dem primären Strömungsweg.

18. Vorrichtung gemäß Anspruch 1, weiterhin umfassend ein Gehäuse.

19. Vorrichtung gemäß Anspruch 18, weiterhin umfassend eine Einrichtung für die Verdrängung von Luft aus der Vorrichtung, wobei die Luft bei Einführung der Flüssigprobe in die Vorrichtung verdrängt wird.

20. Vorrichtung gemäß Anspruch 19, wobei die Einrichtung zum Abführen von Luft mindestens einen Luftauslass umfasst.

21. Vorrichtung gemäß Anspruch 20, umfassend einen Luftauslass, welcher am stromaufwärts gelegenen Ende des sekundären Strömungswegs angeordnet ist.

22. Vorrichtung gemäß Anspruch 21, weiterhin umfassend einen Luftauslass am stromabwärts gelegenen Ende der Vorrichtung.

23. Vorrichtung gemäß Anspruch 4, weiterhin umfassend eine Markierung im sekundären Strömungsweg.

24. Vorrichtung gemäß Anspruch 23, wobei die Bestimmung des Vorhandenseins oder der ungefähren Menge des Analyten in der Flüssigprobe den Nachweis eines optisch nachweisbaren Signals umfasst.

25. Vorrichtung gemäß Anspruch 24, wobei die Markierung ein erstes Verbindungsglied, gekoppelt an einen gefärbten Komponententeil, umfasst und die Markierung ein zweites Verbindungsglied umfasst, und wobei das erste Verbindungsglied zum Binden des zweiten Verbindungsglieds fähig ist.

26. Vorrichtung gemäß Anspruch 25, wobei der in der Flüssigprobe vorhandene Analyt die Markierung bindet und durch das immobilisierte Einfangreagens gebunden wird; und wobei das erste Verbindungsglied, gekoppelt an den gefärbten Komponententeil, an das innerhalb der Markierung umfasste zweite Verbindungsglied gebunden wird, wodurch ein optisch nachweisbares Signal innerhalb der Einfangzone erzeugt wird.

27. Vorrichtung gemäß Anspruch 26, wobei das erste Verbindungsglied und das zweite Verbindungsglied verschieden sind und Biotin bzw. Avidin oder Avidin bzw. Biotin sind.

28. Verfahren zur Bestimmung des Vorhandenseins oder der ungefähren Menge von mindestens einem Analyten in einer Flüssigprobe, umfassend:
Zuführen der Flüssigprobe zu dem ersten Strömungsweg der Vorrichtung gemäß Anspruch 1; und
Bestimmen des Vorhandenseins oder der ungefähren Menge des Analyten in der Einfangzone.

29. Verfahren gemäß Anspruch 28, wobei die Vorrichtung weiter eine Markierungszone umfasst, die stromaufwärts der Einfangzone und stromabwärts der Berührungszone des sekundären Strömungswegs mit dem primären Strömungsweg angeordnet ist.

30. Verfahren gemäß Anspruch 28, wobei der primäre Strömungsweg poröses und nichtporöses Material umfasst.

31. Verfahren gemäß Anspruch 28, wobei die Vorrichtung weiter ein absorptionsfähiges Material stromabwärts der Einfangzone und in Fluidverbindung damit umfasst.

## Revendications

1. Dispositif analytique permettant de réaliser un essai en vue de déterminer la présence ou une quantité approximative d'un analyte dans un échantillon liquide, le dispositif comprenant :
un chemin primaire d'écoulement ;
une zone de capture, capable d'immobiliser l'analyte dans le chemin primaire d'écoulement ; et
un chemin secondaire d'écoulement ;
la seule source de fluide vers le chemin secondaire d'écoulement provenant du chemin primaire d'écoulement, et **caractérisé en ce que** :
le chemin secondaire d'écoulement rejoint le chemin primaire d'écoulement en un seul point de confluence uniquement, en amont de la zone de capture.

2. Dispositif selon la revendication 1, dans lequel le chemin primaire d'écoulement comprend un matériau poreux et un matériau non poreux.

3. Dispositif selon la revendication 1, dans lequel le chemin secondaire d'écoulement est constitué d'un matériau non poreux.

4. Dispositif selon la revendication 1, comprenant en outre :
une zone de marquage située dans le chemin primaire d'écoulement, comprenant au moins un marqueur qui est à même de former un complexe avec l'analyte,
ledit marqueur étant pratiquement mobilisable lorsqu'il est en contact avec l'échantillon liquide ;
la zone de marquage se trouvant en amont d'une zone de capture et en communication fluidique avec cette zone de capture ; et
la zone de capture comprenant un réactif immobilité de capture capable de lier l'analyte.

5. Dispositif selon la revendication 4, dans lequel la zone de marquage se trouve en aval du confluent du chemin secondaire d'écoulement et du chemin primaire d'écoulement.

6. Dispositif selon la revendication 4, dans lequel le chemin primaire d'écoulement comprend du matériau poreux et non poreux et dans lequel la zone de marquage se trouve sur le matériau non poreux et où la zone de capture se trouve sur le matériau poreux ou dans celui-ci.

7. Dispositif selon la revendication 4, dans lequel le chemin primaire d'écoulement comprend du matériau poreux et non poreux, et dans lequel la zone de marquage et la zone de capture se trouvent sur le matériau non poreux ou dans celui-ci.

8. Dispositif selon la revendication 1, comprenant en outre un matériau absorbant en aval de la zone de capture et en communication fluidique avec celle-ci.

9. Dispositif selon la revendication 4, dans lequel le marqueur contient un traceur visible choisi dans l'ensemble constitué d'un fragment coloré, d'un fragment fluorescent et d'un fragment chimioluminescent et d'un fragment phosphorescent.

10. Dispositif selon la revendication 4, dans lequel le marqueur comprend une enzyme, cette enzyme étant à même de réagir avec un substrat d'enzyme pour produire un signal visible.

11. Dispositif selon la revendication 10, dans lequel l'enzyme est choisie dans l'ensemble constitué d'hydrolases, d'estérases et d'oxydoréductases.

12. Dispositif selon la revendication 10, comprenant en outre un réactif de marqueur situé dans le chemin secondaire d'écoulement, ledit réactif de marqueur comprenant un substrat enzymatique.

13. Dispositif selon la revendication 12, dans lequel un signal visible est produit par réaction de l'enzyme avec le substrat enzymatique.

14. Dispositif selon la revendication 1, comprenant en outre une zone de régulation située dans le chemin primaire d'écoulement.

15. Dispositif selon la revendication 1, comprenant en outre une zone de fin d'essai située dans le chemin primaire d'écoulement en aval de la zone de capture.

16. Dispositif selon la revendication 15, dans lequel la zone de fin d'essai comprend un réactif qui produit un signal détectable quand on le met en contact avec l'échantillon.

17. Dispositif selon la revendication 1, comprenant en outre une zone de conditionnement dans le chemin primaire d'écoulement.

18. Dispositif selon la revendication 1, comprenant en outre un boîtier.

19. Dispositif selon la revendication 18, comprenant en outre un moyen servant à déplacer de l'air à partir du dispositif, ledit air étant déplacé lors de l'introduction d'un échantillon liquide dans le dispositif.

20. Dispositif selon la revendication 19, dans lequel le moyen servant à chasser de l'air comprend au moins une bouche d'aération.

21. Dispositif selon la revendication 20, comprenant une bouche d'aération située à l'extrémité amont du chemin secondaire d'écoulement.

22. Dispositif selon la revendication 21, comprenant en outre une bouche d'aération située à l'extrémité aval du dispositif.

23. Dispositif selon la revendication 4, comprenant en outre un traceur dans le chemin secondaire d'écoulement.

24. Dispositif selon la revendication 23, dans lequel la détermination de la présence ou d'une quantité approximative de l'analyte dans l'échantillon liquide comprend la détection d'un signal que l'on peut détecter par voie optique.

25. Dispositif selon la revendication 24, dans lequel ledit traceur comprend un premier élément de liaison couplé à un fragment coloré, et où ledit marqueur comprend un deuxième élément de liaison et où ledit premier élément de liaison est à même de lier ledit deuxième élément de liaison.

26. Dispositif selon la revendication 25, dans lequel l'analyte présent dans l'échantillon liquide se lie audit marqueur et se trouve lié par ledit réactif immobilisé de capture ; et dans lequel ledit premier élément de liaison couplé audit fragment coloré est lié audit deuxième élément de liaison contenu dans ledit marqueur, ce qui produit un signal que l'on peut détecter par voie optique au sein de ladite zone de capture.

27. Dispositif selon la revendication 26, dans lequel ledit premier élément de liaison et ledit deuxième élément de liaison sont différents et sont respectivement la biotine et l'avidine, ou l'avidine et la biotine.

28. Procédé de détermination de la présence ou d'une quantité approximative d'au moins un analyte dans un échantillon liquide, impliquant :
l'écoulement de l'échantillon liquide dans le chemin primaire d'écoulement du dispositif conforme à la revendication 1 ;
et la détermination de la présence ou d'une quantité approximative de l'analyte dans la zone de capture.

29. Procédé selon la revendication 28, ce procédé impliquant en outre une zone de marquage située en amont de la zone de capture et en aval du confluent du chemin secondaire d'écoulement avec le chemin primaire d'écoulement.

30. Procédé selon la revendication 28, dans lequel le chemin primaire d'écoulement comprend un matériau poreux et non poreux.

31. Procédé selon la revendication 28, ce procédé impliquant en outre un matériau absorbant situé en aval de la zone de capture et en communication fluidique avec celle-ci.
